# EUROPEAN PATENT SPECIFICATION

(11) **EP 2 363 117 B1**
(45) Date of publication and mention of the grant of the patent: **19.08.2015**
(21) Application number: 11001973.4
(22) Date of filing: 29.01.2007
(51) Int. Cl.: A61K 9/22, A61K 31/4178

(54) **Drug delivery systems comprising weakly basic selective serotonin 5-HT3 blocking agent and organic acids**
Arzneimittelzubereitung mit schwach basischem selektivem 5-HT3 Blocker und organischen Säuren
Système d'administration de médicaments comportant un agent de blocage de sérotonine 5-HT3 sélective faiblement basique et acides organiques

(30) Priority: 27.01.2006 US 762750 P
(43) Date of publication of application: 07.09.2011
(62) Divisional of application: 07717465.4
(73) Proprietor: Aptalis Pharmatech, Inc., Vandalia, OH 45377 (US)
(72) Inventor: Venkatesh Gopi, M., Vandalia, OH 45377 (US); Lai, Jin-wang, Springboro, OH 45066 (US); Vyas, Nehal, H., Yardley, PA 19067 (US)
(74) Representative: Kremer, Simon Mark

(56) References cited:
- WO-A-2004/096182
- WO-A-2005/077341
- US-A- 6 015 577
- US-A1- 2001 046 964
- US-A1- 2003 113 374
- US-A1- 2003 170 304
- US-A1- 2004 019 096
- US-A1- 2005 238 717

## Description

This application claims the benefit of U.S. Provisional Application No. 60/762,750 filed January 27, 2006.

### TECHNICAL FIELD

The present invention relates to modified-release dosage forms comprising one or more timed, pulsatile-release bead populations comprising a weakly basic nitrogen (N)-containing selective serotonin 5-HT₃ blocking agent having a pKa in the range of from about 5 to 14 and a solubility of not more than 200 µg/mL at a pH of 6.8, and one or more pharmaceutically acceptable organic acids. The dosage form exhibits comparable release profiles of both the active and the organic acid after a predetermined delay (lag time) when dissolution tested by United States Pharmacopoeia (USP) dissolution methodology using a two-stage dissolution medium (first 2 hours in 0.1N HCl followed by testing in a buffer at pH 6.8). In accordance with another aspect of the invention, oral drug delivery systems to target PK (pharmacokinetics, i.e., plasma concentration-time) profiles suitable for a once-daily dosing regimen are disclosed.

### BACKGROUND OF THE INVENTION

Many therapeutic agents are most effective when made available at constant rates at or near the absorption sites. The absorption of therapeutic agents thus made available generally results in desired plasma concentrations leading to maximum efficacy, and minimum toxic side effects. Much effort has been devoted to developing sophisticated drug delivery systems such as osmotic devices for oral application. However, there are instances where maintaining a constant blood level of a drug is not desirable. For example, a major objective of chronotherapy for cardiovascular diseases is to deliver the drug in higher concentrations during the time of greatest need, e.g., the early morning hours, and in lesser concentrations when the need is less, e.g., during the late evening and early sleep hours. In addition to a properly designed drug delivery system, the time of administration is equally important. The unique pharmacokinetic profile needed can be calculated using computer simulation and modeling techniques based on the knowledge of pharmacokinetic parameters, solubility, absorption along the gastrointestinal tract and elimination half-life.

While the orally administered pharmaceutical dosage form passes through the human digestive tract, the drug should be released from the dosage form and be available in solution form at or near the site for absorption from the gastrointestinal (GI) tract to occur. The rate at which the drug goes into solution and is released from a dosage form is important to the kinetics of drug absorption. The dosage form and hence the active ingredient is subjected to varying pHs during the transit, i.e., pH varying from about 1.2 (stomach pH during fasting but may vary between 1.2 and 4.0 upon consumption of food) to about 7.4 (bile pH: 7.0-7.4 and intestinal pH: 5 to 7). Moreover, transit time of a dosage form in individual parts of the digestive tract may vary significantly depending on its size and prevailing local conditions. Other factors that influence drug absorption include physicochemical properties of the drug substance itself such as pKa, solubility, crystalline energy, and specific surface area. The prevailing local conditions that play an important role include properties of luminal contents (pH, surface tension, volume, agitation and buffer capacity) and changes following the ingestion of food. Consequently, it is often difficult to achieve drug release at constant rates.

Basic and acidic drugs exhibit pH-dependent solubility profiles varying by more than 2 orders of magnitude in the physiological pH range. The most difficult candidates to work with are weakly basic pharmaceutically actives, which are practically insoluble at a pH >6 and require high doses to be therapeutically effective. Upon entering into the intestinal region, part of the drug released from the dosage form may precipitate in the hostile pH environment unless the rate of absorption is faster than the rate of drug release. Alternatively, the drug may remain in the supersaturated solution state facilitated by the presence of bile salts and lecithin in the gut. A supersaturation well over an order of magnitude higher than the aqueous solubility has been evident in the prior art. In the event of precipitation, there is evidence of redissolution for absorption at a slower phase.

US2005/0238717 describes an extended release dosage form of ondansetron comprising an amino acid as solubiliser of the drug. The tablet shows a linear release profile over an extended period of time.

Functional polymer membranes comprising suitable combinations of synthetic polymers such as water-soluble (e.g., Povidone), water-insoluble (e.g., ethylcellulose insoluble at physiological pHs), gastrosoluble (e.g., Eudragit EPO) or enterosoluble (e.g., gastric-resistant hypromellose phthalate) polymers, have been applied on tablet or pellet cores comprising the active and one or more solubilizers to achieve drug release at constant rates with limited success. Development of pharmaceutical compositions of actives highly water soluble at acidic or basic pHs using pharmaceutically acceptable buffer acids, buffer acid salts, and mixtures thereof, to provide drug release at substantially constant rates have been described. Organic acids have been used to improve bioavailability, to reduce inter- and intra-subject variability, and to minimize food effect in weakly basic pharmaceutical actives. Multi-particulate dosage forms comprising weakly basic drugs to provide extended-release profiles are also described in the literature. These dosage forms are typically obtained by granulating or layering the drug with one or more organic acids and coating with a combination of water-insoluble and water-soluble or enteric polymers.

Although the drug release in these disclosures could be extended moderately, they suffered from two disadvantages, viz., failure to maintain adequate plasma profile to achieve a once-daily dosing regimen and partial to complete *in situ* formation of the salt form, thus creating a new chemical entity. Even when the organic acid containing cores were coated with a sustained-release polymer membrane, the delivery system failed to prolong the release of the acid for continued dissolution and resulting absorption of the active to provide adequate plasma levels at 24 hrs following oral ingestion. Furthermore, many weakly basic drugs are known to form salts in the presence of organic acids, especially when dissolved in common solvents for drug layering or during granulation. Even in dosage forms wherein the organic acid and the drug layers are separated by a sustained-release (SR) membrane, the drug layering formulation contains an organic acid. Consequently, the active in the finished dosage exists in the partially or fully neutralized salt form. This is not an acceptable situation from regulatory considerations. The regulatory agencies may consider these actives as new drug entities. Thus there is an unmet need to develop drug delivery systems comprising weakly basic drugs with a pKa in the range of from about 5 to 14 and requiring high doses and organic acids in an unaltered form to release the actives so as to maintain target plasma concentrations of Cₘₐₓ and Cₘᵢₙ in order to be suitable for once-daily dosing regimens. After extensive investigations, it was surprisingly discovered that this unmet need can be met by preventing the organic acid and the weakly basic active agent from coming into contact with each other to form a salt during processing and/or in the dosage form during storage, prior to dropping into an *in* vitro dissolution medium or prior to oral administration. This could be achieved by applying a dissolution rate-controlling SR membrane between the acid layer on the inert cores and the drug layer applied onto the acid-containing cores to isolate these two components and also an SR and/or a TPR (lag-time coating) membrane on the IR beads in order to synchronize the acid release with that of the drug.

### SUMMARY OF THE INVENTION

The present invention provides pharmaceutical compositions and methods for creating pulsatile delivery systems, which involves preventing a weakly basic nitrogen (N)-containing selective serotonin 5-HT₃ blocking agent having a pKa in the range of from about 5 to 14 and a solubility of not more than 200 µg/mL at a pH of 6.8, and a pharmaceutically acceptable organic acid from coming into contact to form an acid addition compound. Furthermore, the dosage forms described herein provide target drug-release profiles by solubilizing the drug prior to releasing it into the hostile intestinal environment wherein the drug is practically insoluble, thereby enhancing the probability of achieving acceptable plasma concentration at 24 hour post-dosing in order to be suitable for a once-daily dosing regimen. The invention is particularly useful as disclosed in Provisional Patent Application Ser. No. 60/762,766 to provide dosage forms for a twice- or once-daily dosing regimen of weakly basic nitrogen (N)-containing therapeutic agents having a pKa in the range of from about 5 to 14 (typically soluble at acidic pHs, but poorly to practically insoluble at neutral and alkaline pHs) and an elimination half-life of about 2 hours or longer, by delivering the active in solution form throughout the gastrointestinal tract.

Another embodiment of the invention relates to a multiparticulate pharmaceutical composition containing one or more coated bead populations comprising a weakly basic nitrogen (N)-containing selective serotonin 5-HT₃ blocking agent with a solubility of not more than about 200 µg/mL, more particularly not more than about 100 µg/mL at pH 6.8 and a ratio of optimal highest dose to the solubility at pH 6.8 of at least about 100. For example, the dosing regimen for ondansetron, the active in Zofran^{®} (IR tablet) with a solubility of about 0.05 mg/mL at pH 6.8, is typically 8 mg twice- or thrice-a-day and the optimal highest dose is 16 or 24 mg, the ratio of optimal highest dose (mg) to the solubility (mg/mL) at pH 6.8 would be 320. The multiparticulate composition prepared in accordance with one aspect of the present invention will comprise organic acid-containing cores coated with an SR (sustained-release or barrier) membrane, on which a weakly basic therapeutic agent with a pKa in the range of from about 5 to 14, is layered and further coated with an SR membrane and/or a lag-time membrane such that both the organic acid and the weakly basic therapeutic agent exhibit comparable drug-release profiles.

Multiparticulate compositions prepared in accordance with one aspect of the present invention comprise one or more coated bead populations exhibiting similar composite release profiles of both the organic acid and the weakly basic nitrogen (N)-containing selective serotonin 5-HT₃ blocking agent when tested for dissolution using United States Pharmacopoeia Apparatus 1 (baskets @ 100 rpm) or Apparatus 2 (paddles @ 50 rpm) and a two-stage dissolution methodology (testing in 700 mL of 0.1N HCl (hydrochloric acid) for the first 2 hours and thereafter in 900 mL at pH 6.8 obtained by adding 200 mL of a pH modifier). Another embodiment of the invention relates to a multiparticulate pharmaceutical composition comprising one or more coated bead populations exhibiting the acid-release profile which is more particularly slower in comparison to that of the weakly basic active in order to avoid undissolved active being left behind inside the coated beads.

A multiparticulate pharmaceutical composition in accordance with one aspect of the invention comprises coated bead populations of a weakly basic nitrogen (N)-containing selective serotonin 5-HT₃ blocking agent with a pKa in the range of from about 5 to 14 comprising:
a) an organic acid-containing core particle (organic acid crystal, pellet, bead and the like);
b) a barrier or sustained-release membrane on the acid-containing core particle comprising a water-insoluble polymer or a water-insoluble polymer in combination with a water-soluble or enteric polymer;
c) a weakly basic drug layered on the barrier-coated acid-containing core particle and optionally provided with a protective seal-coat to form an immediate-release (IR) bead;
d) if providing SR beads, an SR coating membrane on the IR bead comprising a water-insoluble polymer or a water-insoluble polymer in combination with a water-soluble polymer forming an SR bead; and / or
e) if providing TPR beads, a lag-time coating membrane on the SR-coated bead comprising a combination of a water-insoluble and enteric polymers to form a timed, pulsatile-release (TPR) bead.

The compositions in accordance with particular aspects of the invention typically exhibit desired or target release profiles of both the active and organic acid following a pre-determined lag-time of at least 2 hours when tested for drug and/or organic acid release using the 2-stage dissolution methodology described above.

A pharmaceutical composition of a weakly basic, nitrogen (N)-containing selective serotonin 5-HT₃ blocking agent with a solubility of not more than about 200 µg/mL at pH 6.8 and a ratio of optimal highest dose to solubility at pH 6.8 of not less than about 100 such as ondansetron hydrochloride dihydrate may be prepared by filling the corresponding bead populations into a hard gelatin capsule or compressing into a conventional tablet or in the ODT (orally disintegrating tablet) form in accordance with certain embodiments of the present invention.

A pharmaceutical composition of a weakly basic nitrogen (N)-containing selective serotonin 5-HT₃ blocking agent in the ODT form prepared in accordance with another embodiment of the present invention disintegrates on contact with saliva in the buccal cavity within about 60 seconds forming a smooth, easy-to-swallow suspension (no gritty or chalky aftertaste). The pharmaceutical composition of a weakly basic pharmaceutical active in the ODT form, which may comprise one or more coated bead populations with an average particle size of not more than about 400 µm, such as taste-masked microcapsules comprising drug-containing cores (crystals, granules, pellets, beads and the like), SR bead and timed, pulsatile-release (TPR) bead populations comprising SR coated acid-containing cores. Taste-masking may be achieved by any of the well-known prior art disclosures. The ODT may also include rapidly-dispersing microgranules with an average particle size of not more than about 400 µm, or in some embodiments not more than about 300 µm, comprising a disintegrant (e.g., Crospovidone, crosslinked polyvinylpyrrolidone) and a sugar alcohol (e.g., mannitol), a saccharide (e.g., lactose) or a combination thereof, each having an average particle size of not more than about 30 µm, and, optionally, pharmaceutically acceptable excipients typically used in ODT formulations, viz., flavors, a sweetener, coloring agents, and additional disintegrant.

The ODT in accordance with one embodiment exhibits the following properties:
1) disintegrates on contact with saliva in the oral cavity in about 60 seconds forming a smooth, easy-to-swallow suspension comprising taste-masked and/or coated particles (SR and/or TPR beads);
2) taste-masked particles, if present, provide rapid, substantially-complete release of the dose upon entry into the stomach (e.g., typically greater than about 50% in about 60 minutes);
3) coated particles (SR and/or TPR beads) provide prolonged release of the active for continued absorption along the GI tract.

The ODT in accordance with one embodiment comprising taste-masked microparticles demonstrating effective taste-masking by releasing not more than 10% in about 3 minutes (the longest typical residence time anticipated for the ODT in the buccal cavity) when dissolution tested in simulated saliva fluid (pH ~6.8) while releasing not less than about 50% of the dose in about 60 minutes when dissolution tested in 0.1N HCl.

In accordance with certain embodiments, the rapidly-dispersing microgranules and coated beads (taste-masked IR, and SR and/or TPR beads) of one or more weakly basic actives may be present in the weight ratio of about 6:1 to 1:1, more particularly from about 4:1 to 2:1, to achieve a smooth (non-gritty) mouth feel. In accordance with certain other embodiments, the coated beads (taste-masked IR, and SR and/or TPR beads) of one or more weakly basic actives may be coated with a compressible coating (e.g., fluid-bed coating with a plasticized aqueous dispersion of ethylcellulose) in order to minimize membrane fracture during compression with rapidly-dispersing microgranules.

A pharmaceutical composition of a weakly basic pharmaceutical active in the conventional tablet form in accordance with another embodiment of the present invention, may comprise one or more bead populations, such as IR beads (crystals, granules, pellets, beads and the like), and SR beads and/or TPR beads comprising SR coated acid-containing cores. The pharmaceutical composition of a weakly basic pharmaceutical active in the conventional tablet form disintegrates into constituent beads (taste-masked particles, coated SR beads and/or TPR beads) upon oral ingestion in about 10 minutes. The conventional tablet may also include pharmaceutically acceptable excipients typically used in disintegrating tablet formulations such as compressible diluents, fillers, coloring agents, and optionally a lubricant.

The conventional tablet prepared in accordance with one embodiment exhibits the following properties:
1) disintegrates upon oral ingestion in about 10 minutes into IR particles and/or coated particles (SR and/or TPR beads);
2) IR particles, if present, provide rapid, substantially-complete release (e.g., greater than about 95%) of the dose within about 60 minutes, more particularly within about 30 minutes upon entry into the stomach;
3) SR and/or TPR beads provide prolonged release of the active for continued absorption along the gastrointestinal (GI) tract

Another embodiment of the invention relates to a multiparticulate pharmaceutical composition comprising one or mote coated bead populations comprising one or more weakly basic therapeutic agents having an elimination half-life of about 2 hours or longer, wherein the active is layered onto SR coated organic acid-containing cores. The pulsatile delivery system developed in accordance with this aspect of the present invention may comprise IR bead, SR bead and timed, pulsatile-release (TPR) bead populations. The SR coated organic acid-containing cores are typically prepared by layering an organic acid (e.g., fumaric acid) onto inert particles (e.g., sugar spheres) from a polymeric binder solution and coated with a water-insoluble polymer (e.g., ethylcellulose, with a viscosity of about 10 cps) alone or in combination with a water-soluble polymer (e.g., polyvinylpyrrolidone, Povidone K-25 or polyethylene glycol, PEG 400) or an enteric polymer (e.g., hypromellose phthalate, HPMCP or HP-55). The IR bead population comprising SR coated acid-containing cores are prepared by drug layering onto SR coated acid-containing cores from a polymeric binder solution and providing a protective seal coat of Opadry Clear. The SR and TPR bead populations are prepared by coating IR beads with a water-insoluble polymer (e.g., ethylcellulose) alone or in combination with a water-soluble polymer (e.g., PVP K-25 or PEG 400). In accordance with one aspect of the invention each SR or TPR bead population releases both the drug and the acid at comparable rates, as rapid-release or sustained-release profiles after a pre-determined lag-time (for example, a lag-time of up to 10 hours) upon oral administration. IR beads, if included in the dosage form (capsule or conventional tablet or orally disintegrating tablet), may comprise the drug layered directly onto inert cores and coated with a protective seal coat or a taste-masking membrane, which being part of the total dose, provides for rapid absorption (a bolus dose) upon oral administration.

A method of manufacturing a multiparticulate pharmaceutical composition wherein a delivery system developed in accordance with certain embodiments of the present invention comprises one or more weakly basic active pharmaceutical ingredients in sufficient quantities to be administered orally to a patient at prescribed once-daily dosing regimen to provide therapeutic efficacy is also provided.

The method of manufacturing a multiparticulate pharmaceutical composition in accordance with particular embodiments includes layering of a pharmaceutically acceptable organic acid such as fumaric acid from a polymeric binder solution onto inert particles selected from the group consisting of sugar spheres and cellulose spheres. Fluid bed or pan coating may be used to apply the organic acid and polymeric binder solution. In accordance with other embodiments, the core particles may be crystals with a desired particle size distribution, microgranules, pellets or beads containing one or more organic acid(s). In accordance with certain embodiments, the microgranules, extruded-spheronized pellets or compressed microtablets comprising one or more organic acids, a polymeric binder, which imparts resilient characteristics to dried microgranules, hydrophilic fillers/diluents, and optionally a flavor, a sweetener and/or a disintegrant. These organic acid-containing particles are barrier coated with an SR (sustained release) polymer membrane comprising a water-insoluble polymer (e.g., ethylcellulose with an average viscosity of 10 cps) alone or in combination with a water-soluble polymer (e.g., polyvinyl pyrrolidone or polyethylene glycol) or an enteric polymer (e.g., hypromellose phthalate (HPMCP or HP-55)). The water-insoluble and water-soluble or enteric polymers may be present at a weight ratio of from about 95:5 to about 50:50, more particularly from about 90:10 to 60:40 and the membrane thickness may vary from about 3% to 50%, more particularly from about 5% to 30% by weight in accordance with particular embodiments.

In accordance with particular embodiments, one or more weakly basic drug(s) are applied onto SR coated acid-containing particles from a polymeric binder solution and also, a protective seal coat with a hydrophilic polymer (e.g., Pharmacoat^{™} 603 or Opadry^{®} Clear) is applied on drug-layered beads to produce IR beads. The organic acid or drug load depends on the physicochemical as well as the pharmacological properties of the weakly basic actives chosen for development, and the drug and the organic acid may be present at a weight ratio of from about 5:1 to 1:10 or more particularly from about 3:1 to 1:3 depending on whether organic acid crystals or organic acid-containing cores are used in. accordance with certain embodiments.

In accordance with certain embodiments of the present invention, the IR beads comprising SR coated acid-containing cores are barrier coated with an SR polymer membrane comprising a water-insoluble polymer (e.g., ethylcellulose with an average viscosity of 10 cps) alone or in combination with a water-soluble polymer (e.g., polyvinyl pyrrolidone or polyethylene glycol). The water-insoluble and water-soluble polymers may be present at a weight ratio of from about 95:5 to about 50:50, more particularly from about 90:10 to 60:40 and the membrane thickness may vary from about 3% to 50%, more particularly from about 5% to 30% by weight in accordance with particular embodiments.

In accordance with other embodiments of the present invention, the SR beads comprising drug-layered beads are coated with a lag-time membrane comprising a combination of a water-insoluble polymer (e.g., ethylcellulose with an average viscosity of 10 cps) and an enteric polymer (e.g., hypromellose phthalate (HPMCP or HP-55)) to produce TPR beads. In accordance with certain other embodiments, the water-insoluble and enteric polymers may be present at a weight ratio of from about 9:1 to about 1:4, more particularly from about 3:1 to 1:1, and the membrane thickness may vary from about 5% to 60%, more particularly from about 15% to 50% by weight in accordance with particular embodiments.

The functional polymeric systems being applied from aqueous or solvent-based compositions typically contain plasticizers at suitable concentrations. The finished dosage form may be a modified-release (MR) capsule, a standard (conventional) tablet or an orally disintegrating tablet (ODT) comprising a coated spherical bead population containing the active substance alone or a combination of two or more coated bead populations to provide target plasma concentrations suitable for a once-daily dosing regimen. For example, a once-daily dosage form of an active with an elimination half-life of about 7 hours may contain a mixture of an IR bead population which allows immediate release, a second, TPR bead population with a shorter lag-time (about 3-4 hours), which allows a delayed, rapid - release and a third TPR bead population with a longer lag-time (about 7-8 hours), which allows typically a delayed, sustained-release profile over about 8-12 hours, to maintain acceptable plasma concentrations at 24 hrs, thus enhancing safety, therapeutic efficacy and patient compliance while reducing cost of treatment. Alternatively, the finished dosage form may comprise an IR bead population and a second, TPR bead population with a lag-time of about 7-8 hours followed by a sustained-release profile over 10-12 hours. The achievable lag time depends on the composition and thickness of the barrier coating, as well as the composition and thickness of the lag-time coating. Specific factors that can affect achieving optimal once-daily dosage forms include, but are not limited to, the therapeutic agent's pKa (and its solubility above a pH of 6.0), elimination half-life, and solubility enhancement in an aqueous solution of an organic acid selected from the group consisting of aspartic acid, citric acid, fumaric acid, maleic acid, oxalic acid, succinic acid, tartaric acid, and the like.

In accordance with certain embodiments of the present invention, a method of manufacturing a multiparticulate composition comprising a weakly basic nitrogen (N)-containing selective serotonin 5-HT₃ blocking agent having a pKa in the range of from about 5 to 14 and a solubility of not more than 200 µg/mL at a pH of 6.8 is also provided. The method may comprise the steps of:
a) preparing core particles (crystals with a particle size distribution of 20-500 µm, more particularly of 100-300 µm, beads or pellets) of one or more pharmaceutically acceptable organic acids;
b) coating these acid-containing cores with a water-insoluble polymer or a water-insoluble polymer in combination with a water-soluble or enteric polymer in order to program the release of the acid for a weight gain of from about 3% to 50%;
c) layering said weakly basic nitrogen (N)-containing selective serotonin 5-HT₃ blocking agent from a polymeric binder solution and applying a protective seal-coat onto the drug-layered beads to produce IR beads;
d) applying a barrier (sustained-release) coating of a water-insoluble polymer or a water-insoluble polymer in combination with a water-soluble polymer for a weight gain of from about 3% to 30% to produce SR beads;
e) applying a lag-time (time-delay) coating of a combination of water-insoluble and enteric polymers at a weight ratio of from about 10:1 to 1:4 for a weight gain of from about 10% to 60% by weight of the coated bead to produce TPR beads; and
f) filling into hard gelatin capsules or compressing into conventional tablets/ orally disintegrating tablets (ODTs) after blending with pharmaceutically acceptable excipients, one or more bead populations (e.g., a combination of IR beads, SR beads and/or TPR beads at a desired ratio).

The composition comprising one or more bead populations (e.g., a combination of IR and TPR bead populations) may exhibit the following properties:
a) the composition disintegrates on contact with saliva in the oral cavity forming a smooth, easy-to-swallow suspension (if in the ODT form) or disintegrates within about 10 minutes upon oral ingestion (if in the conventional tablet or capsule form);
b) the IR beads, if taste-masked, rapidly releases of the dose upon entry into the stomach (e.g., typically greater than about 50%, more particularly greater than about 75%, in about 60 minutes);
c) the SR or TPR beads releasing the drug over a period of about 4 to 20 hours in synchronization with that of the organic acid after a predetermined delay (e.g., up to about 10 hours) following oral administration;
d) the composite drug-release profile of the composition is similar to target *in vitro* drug-release / *in vivo* plasma concentration profile in order to be suitable for a once-daily dosing regimen.

These and other embodiments, advantages and features of the present invention become clear when detailed descriptions and examples are provided in subsequent sections.

### BRIEF DESCRIPTION OF THE DRAWINGS

FIG. 1 illustrates pH-solubility profiles for (a) Ondansetron hydrochloride, (b) Carvedilol, (c) Dipyridamole, and (d) Clonazepam.

FIG. 2 illustrates a cross-section of an SR coated organic acid-containing core in accordance with one aspect of the invention.

FIG. 3 illustrates a cross-section of a TPR bead comprising an SR coated organic acid-containing core in accordance with a particular aspect of the invention.

FIG. 4 illustrates the release of fumaric acid from SR-coated acid crystals of Example 1 A.

FIG. 5 illustrates the release of the acid and ondansetron hydrochloride from TPR beads of Example 1C.

FIG. 6 illustrates the simulated plasma concentration - time profiles of ondansetron hydrochloride MR formulation one-daily (qd) versus actual 8 mg ondansetron hydrochloride IR tablet tid.

FIG. 7 illustrates the release profiles of ondansetron hydrochloride from TPR beads of Example 3.

FIG. 8 shows the release profiles of both fumaric acid and ondansetron hydrochloride from SR beads (lot# 1084-060) coated with 60/40 EC-10/PEG 400 at 5 and 10% of Example 3.

FIG. 9 illustrates the release profiles of ondansetron hydrochloride from TPR beads of Example 4.

FIG. 10 illustrates the release profiles of ondansetron hydochloride from MR capsules comprising IR and TPR beads at a ratio of 35/65 by weight of Example 5.

### DETAILED DESCRIPTION OF THE INVENTION

All documents cited are, in relevant part, incorporated herein by reference; the citation of any document is not to be construed as an admission that it is prior art with respect to the present invention.

As used herein, as well as in specific examples thereof, the term "weakly basic pharmaceutical active" includes the base, pharmaceutically acceptable salts, polymorphs, stereoisomers and mixtures thereof. This term, which is more fully defined in a subsequent section, refers to a nitrogen (N)-containing selective serotonin 5-HT₃ blocking agent having a pKa in the range of from about 5 to 14 and a solubility of not more than 200 µg/mL at a pH of 6.8 and a ratio of optimal highest dose to solubility at pH 6.8 of not less than about 100.

As used herein, the term "immediate release" refers to release of greater than or equal to about 50% (especially if taste-masked for incorporation into an orally disintegrating tablet dosage form), preferably greater than about 75%, more preferably greater than about 90%, and in accordance with certain embodiments greater than about 95% of the active within about 2 hours, more particularly within about one hour following administration of the dosage form. The term can also refer to the release of the active from a timed, pulsatile release dosage form characterized by an immediate release pulse after the designed lag time. The term "lag-time" refers to a time period wherein less than about 10%, more particularly substantially none, of the dose (drug) is released, and a lag-time of from at least about 2 to 10 hours is achieved by coating typically with a combination of water-insoluble and enteric polymers (e.g., ethylcellulose and hypromellose phthalate).

Unless indicated otherwise, all percentages and ratios are calculated by weight based on the total composition.

An aqueous or a pharmaceutically acceptable solvent medium may be used for preparing organic acid-containing core particles for drug layering, viz., acid-containing beads by layering an acid onto inert cores (e.g., sugar spheres) or IR beads by drug-layering onto acid-containing cores or directly onto sugar spheres from an appropriate polymer binder solution in fluid-bed equipment. Also, an aqueous dispersion of functional polymers, which are available as dispersions or a solvent system may be used for dissolving functional polymers for coating acid-containing beads, IR beads or SR beads.

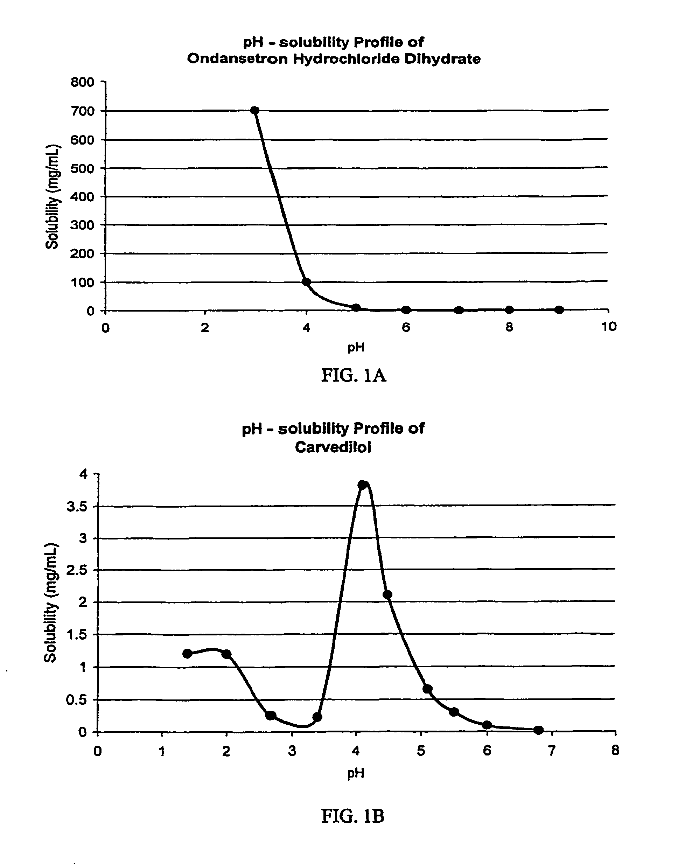

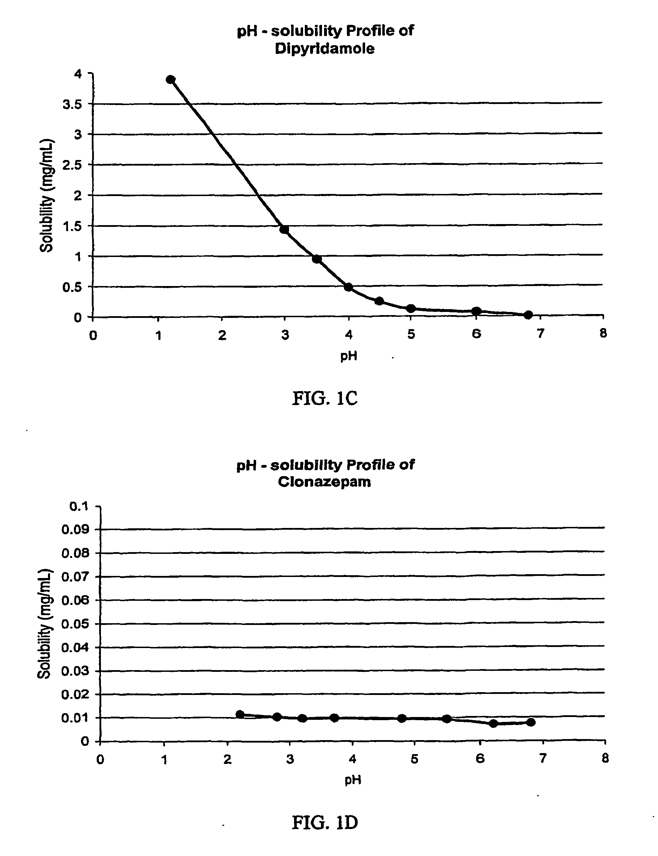

Many active pharmaceutical ingredients (API) are weakly basic in the sense that these actives are freely to moderately soluble at acidic pHs, but are poorly to practically insoluble at neutral and alkaline pHs. Their pKa values are in the range of about 5 to 14. The pH-dependent solubility data for typical weakly basic actives are presented in Fig. 1. For example dipyridamole's solubility in 0.1N HCl (hydrochloric acid) is about 1 mg/mL while at pH 6.8, the solubility is only 30 µg/mL. Although carvedilol's solubility is similarly pH-dependent and varying, it is not obvious from Fig. 1 as it rapidly undergoes in *situ* salt formation with the buffering agent such as citric, acetic, and hydrochloric acids and consequently, the observed solubility is that of the salt formed *in-situ.*

Table 1 lists the solubility enhancement of weakly basic actives in organic acid buffers. Three distinct groups can be identified. Group A actives, as represented by ondansetron hydrochloride, exhibits a dramatic increase in solubility of the weakly basic active in a buffer with a trace of fumaric acid. For example, ondansetron's solubility of about 26 µg/mL in the buffer containing only 0.05 mg/mL of fumaric acid remains unchanged upon increasing the concentration of fumaric acid in the buffer up to 5 mg/mL. In Group B, represented by dipyridamole, carvedilol and lamotrigine, the weakly basic drug's

**Table 1: Solubilities of Weakly Basic Drugs in Organic Acids**

| Concentration of Fumaric Acid (mg/mL) | Start pH | End pH | Solubility of Ondansetron Hydrochloride (mg/mL) | Start pH | Solubility of Dipyridamole (mg/mL) |
|---|---|---|---|---|---|
| 5 | 2.13 | 2.01 | 26.9 | 2.98 | 6.24 |
| 2.5 | 2.26 | 2.14 | 27.0 | 3.42 | 1.80 |
| 1 | 2.48 | 2.40 | 26.1 | 3.68 | 0.93 |
| 0.25 | 2.79 | 2.75 | 26.2 | 3.88 | 0.65 |
| 0.05 | 3.19 | 3.49 | 26.0 | 4.33 | 0.27 |
| 0.01 | 3.64 | 4.05 | 26.1 | 4.71 | 0.13 |
| 0.0025 | 4.15 | 4.33 | 26.1 | 6.28 | 0.006 |

| Solubility (mg/mL) of Carvedilol in Tartaric Acid | | Solubility (mg/mL) of Lamotrigine in Tartaric Acid | | Solubility (mg/mL) of Clonazepam in Fumaric Acid | |
|---|---|---|---|---|---|
| pH of Buffer | (mg/mL) | pH of Buffer | (mg/mL) | pH of Buffer | (mg/mL) |
| 2.12 | 2.51 | 2.43 | 4.48 | 2.3 | 0.0116 |
| 2.28 | 1.36 | 3.33 | 1.77 | 2.8 | 0.0103 |
| 2.54 | 0.731 | 4.36 | 1.61 | 3.2 | 0.0096 |
| 2.94 | 0.508 | 4.97 | 0.488 | 3.7 | 0.0098 |
| 3.64 | 0.121 | 5.66 | 0.226 | 4.8 | 0.0095 |
| 5.46 | 0.105 | 5.85 | 0.197 | 5.5 | 0.0093 |
| 5.90 | 0.028 | 6.50 | 0.161 | 6.2 | 0.0072 |
| | | | | 6.8 | 0.0069 |

solubility increases with increasing concentration of the acid. In Group C, represented by clonazepam, the organic acid has very limited impact, i.e., the solubility enhancement amounts typically to less than 3-fold. For example, clonazepam's solubilities are about 11.6 and 6.9 µg/mL in buffers at pH 2.3 and 6.8 containing a higher and lower concentration of fumaric acid, respectively.

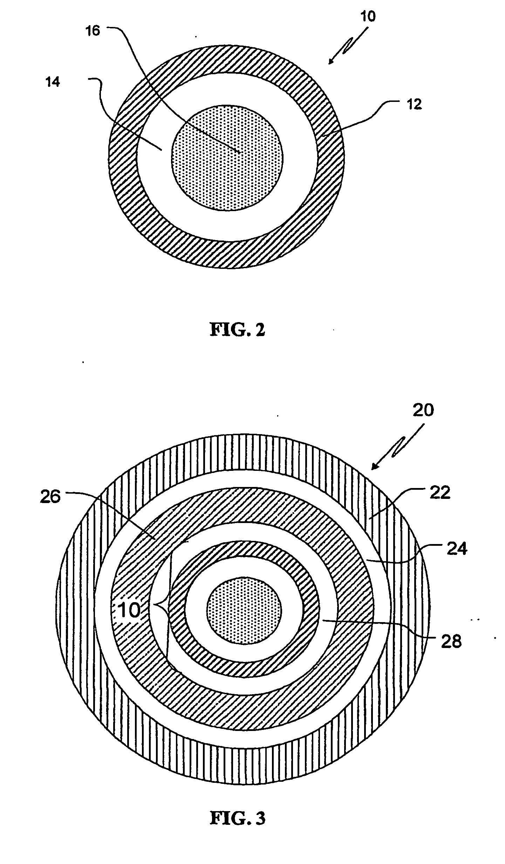

Specific embodiments of the invention will be described in further detail with reference to the accompanying Figures 2 and 3. In Fig. 2, an SR-coated core 10 comprising an SR coating 12 applied on an organic acid-containing core comprising a layer of a pharmaceutically acceptable organic acid in a binder 14 coated on an inert particle core 16. The inert particle core 16, organic acid-coating layer 14 and a dissolution rate controlling SR layer 12 make up the SR-coated organic acid-containing core 10. In Fig. 3, a representative TPR bead is illustrated. The TPR bead 20 comprises a lag-time coating 22 applied on a primary SR layer 24, a protective seal-coat 26 and a weakly basic drug layer 28 applied on an SR-coated acid-containing core 10. The weakly basic drug is typically applied from a polymeric binder solution. The SR coating sustains the drug release while the lag-time coating provides the lag-time (a time period exhibiting less than about 10%, more particularly substantially none, of the dose released). Thus the lag-time coating 22, outer SR coating on the IR beads 24, and inner SR coating 12 on the acid-containing core together control the release properties of both the drug and acid from the TPR beads.

The novelty/utility of the formulations developed in accordance with certain embodiments of the present invention is disclosed using ondansetron hydrochloride as an example of weakly basic nitrogen (N)-containing selective serotonin 5-HT₃ blocking agents having a pKa in the range of from about 5 to 14. Ondansetron hydrochloride dihydrate is chemically (±) 1, 2, 3, 9-tetrahydro-9-methyl-3-[(2-methyl-1H-imidazole-1-yl) methyl]-4H-carbazol-4-one monohydrochloride dihydrate. Ondansetron is indicated for the prevention of nausea and vomiting associated with radiotherapy and/or chemotherapy and prevention of postoperative nausea and/or vomiting. Zofran® Tablets (Ondansetron HCl Dihydrate, 4, 8, and 24 mg base equivalent) are commercially available. Drug is administered 8 mg bid for chemotherapy and 8 mg tid for radiotherapy. A once-daily dosing of ondansetron hydrochloride is commercially desirable and would simplify the dosing regimen and enhance patient compliace. Ondansetron exists as a racemate and it contains an α-hydroxyl secondary amine, with a pKa of 7.4. Ondansetron HCl is known to exhibit a pH-dependent solubility profile (solubility decreasing by 2-3 orders of magnitude). Ondansetron is well absorbed from the gastrointestinal tract and undergoes some first-pass metabolism. The elimination half-life averages approximately 3.8±1 hrs. Since the drug dissolution is the rate-limiting factor for absorption in the distal part of the GI tract potentially due to the decrease in solubility, the once-daily dosage form in accordance with one embodiment would comprise at least two bead populations - one IR bead population and another TPR bead population comprising SR coated organic acid cores.

In accordance with certain embodiments of the present invention, the solubility enhancing property of organic acid buffers is taken advantage of, and at the same time, the in *situ* formation of acid addition compounds is prevented by having an SR coating membrane between the inner organic acid layer and the weakly basic drug layer. The SR coating membrane thus applied precisely controls the release of the organic acid so as to insure no drug is left behind in the dosage form for lack of solubilizing acid in the TPR bead. In one embodiment, the active core of the dosage form of the present invention may comprise an inert particle coated with an organic acid, an SR coating, drug-layered (IR beads), further barrier or SR coated and/or lag-time coated. The amount of organic acid and the drug-load in the core will depend on the drug, the dose, its pH-dependent solubility, solubility enhancement, and elimination half-life. Those skilled in the art will be able to select an appropriate amount of drug/acid for coating onto the core to achieve the desired QD (once-daily) dosing regimen. In one embodiment, the inert particle may be a sugar sphere, a cellulose sphere, a silicon dioxide sphere or the like. Alternatively, organic acid crystals with a desired particle size distribution may function as cores, especially for Group C drugs, and in this case, these crystals are membrane coated to program the acid release, which, in accordance with certain embodiments, is synchronized with that of the drug to ensure complete release of the drug prior to depletion of the acid.

In accordance with one aspect of the present invention, the core of the dosage form may comprise an organic acid (e.g., fumaric acid) crystal with a desired mean particle size or an inert particle such as a sugar sphere layered with an organic acid from a polymer binder solution. Organic acid crystals or acid-containing cores are coated with a water-insoluble polymer alone or in combination with a water-soluble or enteric polymer, and the composition and thickness of the SR membrane is optimized such that the acid release is slower than or synchronized with the drug dissolution/release from the bead, thereby ensuring that the acid release is not complete prior to depletion of the drug release. In certain aspects of the invention, the acid-containing cores may be in the form of microgranules or pellets which may be prepared by rotogranulation, high-shear granulation and extrusion-spheronization or compression (as micro-tablets about 1-1.5 mm in diameter) of the organic acid, a polymeric binder and optionally fillers/diluents.

A weakly basic active agent such as ondansetron hydrochloride dihydrate is layered onto the SR coated fumaric acid-containing beads from a polymeric binder (e.g., povidone) solution and a protective seal-coat comprising a hydrophilic polymer such as Pharmacoat 603 (Hypromellose 2910 3 cps) or Opadry® Clear to form IR beads. In one embodiment, the drug-containing IR beads may be coated twice - an inner barrier coating membrane with a water-insoluble polymer (e.g., ethylcellulose) alone or in combination with a water-soluble polymer and a lag-time coating membrane of a water-insoluble polymer in combination with an enteric polymer to produce TPR beads with a lag-time (release with a delayed-onset) of approximately 1 to 10 hours upon oral administration. The water-insoluble polymer and enteric polymer may be present at a weight ratio of from about 9:1 to about 1:4, preferably at a weight ratio of from about 3:1 to 1:1. The membrane coating typically comprises from about 5% to about 60%, preferably from about 10% to about 50% by weight of the coated beads. In accordance with yet another embodiment, the IR beads may simply be coated with a combination of a water-insoluble polymer and an enteric polymer in the aforementioned amounts.

The unit capsule or conventional tablet dosage form according to the present invention may comprise TPR beads alone or in combination with IR beads while the unit ODT may comprise TPR beads alone or in combination with taste-masked immediate release (IR) beads. IR beads without having a taste-masking membrane will provide rapid release of the weakly basic drug in the gastrointestinal tract within approximately 60 minutes, preferably within 30 minutes following oral administration. If taste-masked, these beads exhibit taste-masking in the buccal cavity and substantially complete release of the weakly basic drug in the gastrointestinal tract within approximately 2 hours, preferably within one hour following oral administration. The TPR beads will release the weakly basic drug over a period of up to approximately 4-20 hours in the gastrointestinal tract after a lag time of about 1-10 hours following oral administration.

In accordance with particular aspects of the present invention, the pharmaceutical multiparticulate dosage form may comprise at least an IR bead population, a first TPR bead population, and an SR bead population or a second TPR bead population. In certain embodiments, the ratio of IR bead population to the first TPR bead population to the SR bead or second TPR bead populations may vary from about 10:90:0 to about 40:10:50.

The present invention also provides a method for manufacturing a pharmaceutically elegant multiparticulate dosage form having one or more timed_{;} pulsatile release bead populations of one or more weakly basic actives comprising SR-coated organic acid-containing cores, i. e., a well time-controlled, series of pulses so that the active agents and the acid, being deposited in well separated/isolated layers, do not come into contact with each other to form acid-addition compounds until the dosage form comes into contact with a dissolution medium or body fluids following oral ingestion. The dosage form thus produced exhibits composite release profiles of the active agent and the acid that are comparable, more particularly, the acid-release profile is slower than that of the drug so that no undissolved drug is left behind in the dosage form for lack of solubilizing organic acid.

In accordance with one embodiment of the present invention, the method may include the steps of:
a. providing an organic acid-containing core particle (e.g., an organic acid crystal with a desired particle size distribution or a particle comprising an inert particle (e.g., a sugar sphere, a cellulose sphere, a silicon dioxide sphere) layered with an organic acid from a polymeric binder solution);
b. coating the organic acid-containing core particle with an SR coating membrane consisting of a water-insoluble polymer such as EC-10 (ethylcellulose with a mean viscosity of 10 cps) alone or in combination with a water-soluble polymer (e.g., povidone or PEG 400) or an enteric polymer such as hydroxypropyl methylcellulose phthalate (e.g., HP-55);
c. applying a layer of a weakly basic drug such as ondansetron hydrochloride dihydrate onto the SR coated organic acid-containing core particle and further applying a protective seal-coat of Pharmacoat 603 or Opadry® Clear to form an IR bead;
d. applying a barrier coating membrane onto the IR bead with a solution of a water-insoluble polymer (e.g., ethylcellulose) alone or in combination with a water-soluble polymer (e.g., polyethylene glycol, PEG 400) to produce an SR bead;
e. applying a lag-time coating membrane onto the SR bead with a solution of a water-insoluble polymer in combination with an enteric polymer (e.g., ethylcellulose and hypromellose phthalate) at a ratio of about 10:1 to 1:4 to form a timed pulsatile-release drug particle (TPR) bead.

In accordance with certain embodiments of the present invention, the method may include the steps of
i. taste-masking IR beads by solvent coacervation with a water-insoluble polymer (e.g., ethylcellulose with a mean viscosity of 100 cps) alone or in combination with a gastrosoluble pore-former (e.g., calcium carbonate) in accordance with the disclosure in the co-pending US Patent Application Ser. No. 11/213,266 filed Aug. 26, 2005 (Publication No. U.S. 2006/0105038 published May 18, 2006) or by fluid-bed coating with a water-insoluble polymer (e.g., ethylcellulose with a mean viscosity of 10 cps) alone or in combination with a gastrosoluble polymer (e.g., Eudragit E100 or EPO) in accordance with the disclosure in the co-pending US Patent Application Ser. No. 11/248,596 filed Oct. 12, 2005 (Publication No. U.S. 2006/0078614 published April 13, 2006) or a gastrosoluble pore-former (e.g., calcium carbonate) in accordance with the disclosure in the co-pending US Patent Application Ser. No. 11/256,653 filed Oct. 21, 2005 (Publication No. U.S. 2006/0105039 published May 18,2006).
ii. granulating a powder mixture of a sugar alcohol such as mannitol or a saccharide such as lactose and crospovidone, for example, using the disclosure in the co-pending US Patent Application Ser. No. 10/827,106 filed Apr. 19, 2004 (Publication No. U.S. 2005/0232988 published October 20, 2005), to produce rapidly-dispersing microgranules;
iii. blending one or more TPR bead populations from step (e) alone or in combination with taste-masked IR beads from step (i), and/or SR beads from step (d) at a desired ratio to provide a desired once-daily plasma profile, rapidly-dispersing microgranules from step (ii) and other pharmaceutically acceptable excipients; and
iv. compressing the blend from step (iii) into orally disintegrating tablets comprising required dose of one or more weakly basic drugs, which would rapidly disintegrate on contact with saliva in the buccal cavity forming a smooth, easy-to-swallow suspension and exhibiting a plasma profile suitable for a once-daily dosing regimen with reduced incidence of adverse events including non-compliance.

An aqueous or a pharmaceutically acceptable solvent medium may be used for preparing core particles based on coated inert particles. The type of inert binder that is used to bind the water-soluble organic acid or weakly basic drug to the inert particle or to the SR coated acid-containing core is not critical but usually water soluble or alcohol soluble binders, such as polyvinylpyrrolidone (PVP or povidone) or hydroxypropylcellulose may be used. The binder may be used at any concentration capable of being applied to the inert particle. Typically, the binder is used at a concentration of about 0.5 to 10% by weight. The organic acid or the weakly basic drug may be preferably present in this coating formulation in solution form. The drug concentration may vary depending on the application but typically will be used at concentrations from about 5 to 30% by weight depending on the viscosity of the coating formulation.

In accordance with other embodiments, the organic acid-containing cores may be prepared by rotogranulation, or by granulation followed by extrusion-spheronization or tableting into micro-tablets. The organic acid, a binder, and optionally other pharmaceutically acceptable excipients (e.g., diluents/fillers) may be blended together in a high-shear granulator, or a fluid bed granulator, such as Glatt GPCG granulator, and granulated to form agglomerates. The wet mass can be extruded and spheronized to produce spherical particles (pellets). The blend comprising acid particles, a binder and optionally a filler/diluent or drug-containing granules can also be compressed into micro-tablets (about 1-1.5 mm in diameter) to produce organic acid-containing pellets. In these embodiments, the acid content could be as high as 95% by weight based on the total weight of the granulated, extruded or compressed core. These acid-containing cores are coated with an SR membrane prior to drug-layering and subsequent coating with functional polymers.

The individual polymeric coatings on the acid-containing cores and IR beads will vary from about 5 to 50% by weight depending on the relative solubility of organic acid to active, nature of the active, composition of the barrier coat, and required lag-time. In one embodiment, the acid cores may be provided with a barrier-coat of a plasticized water-insoluble polymer, such as ethylcellulose (EC-10), at about 5-50% by weight to sustain the acid release over about 5-20 hours. In certain other embodiments, the acid cores may be provided with a barrier-coat of a plasticized ethylcellulose and hydroxypropyl methylcellulose (hypromellose) phthalate (HP-55) at about 10-50% by weight while the IR beads are coated with ethylcellulose (EC-10) at 5-20% by weight to achieve the drug-release synchronized with that of the acid. In yet another embodiment of the present invention, the IR beads may not be provided with any barrier coating, and the outer lag-time coating of EC-10/HP-55/plasticizer at about 45.5/40/14.5 for a weight gain of about 30-50% by weight controls the drug-release following the lag-time. The composition of the membrane layer and the individual weights of the polymers are important factors to be considered for achieving a desired drug/acid-release profile and lag time prior to appreciable drug release.

The drug/acid-release profiles from IR beads, barrier/SR-coated beads and TPR beads may be determined according to the following procedure:

Dissolution testing of IR beads, taste-masked or not, is conducted with a USP Apparatus 1 (baskets at 100 rpm) or Apparatus 2 (paddles at 50 rpm) in 900 mL of 0.1N HCl at 37°C while the dissolution testing of SR and TPR beads is conducted in a USP apparatus using a two-stage dissolution medium (first 2 hours in 700 mL of 0.1N HCl at 37°C followed by dissolution testing at pH = 6.8 obtained by the addition of 200 mL of a pH modifier). Drug/acid-release with time is determined by HPLC on samples pulled at selected intervals.

There are instances wherein the onset of drug release should begin several hours following oral administration to provide adequate plasma concentration to be suitable for a once-daily dosing regimen, depending on the elimination half-life of the active. In accordance with particular aspects of the invention, drug release may be delayed for up to about 8-10 hours after oral administration.

A single targeted sustained-release profile over several hours after oral administration, with or without an immediate release pulse, is provided in accordance with certain embodiments of the present invention.

An aqueous or a pharmaceutically acceptable solvent medium may be used for preparing organic acid-containing core particles or drug-containing IR Beads by layering the drug onto inert cores such as sugar spheres or onto SR-coated acid-containing cores. The type of inert binder that is used to bind the water-soluble organic acid to the inert particle or the weakly basic drug onto SR-coated acid cores is not critical but usually water-soluble or alcohol and/or acetone-soluble binders are used. Representative examples of binders include, but are not limited to, polyvinylpyrrolidone (PVP), hydroxypropyl methylcellulose (HPMC), hydroxypropylcellulose, carboxyalkylcelluloses, polyethylene oxide, polysaccharides such as dextran, corn starch, which may be dissolved or dispersed in water, alcohol, acetone or mixtures thereof. The binders are typically used at a concentration of from about 0.5 to 10% by weight

Representative inert particles used to layer the acid or the pharmaceutical active include sugar spheres, cellulose spheres and silicon dioxide spheres with a suitable particle size distribution (e.g. 20-25 mesh sugar spheres for making coated beads for incorporation into a capsule formulation and 60-80 mesh sugar spheres for making coated beads for incorporation into an ODT formulation).

Representative pharmaceutically acceptable organic acids which enhance the solubility of the pharmaceutical active include citric acid, fumaric acid, malic acid, maleic acid, tartaric acid, succinic acid, oxalic acid, aspartic acid, glutamic acid and the like. The ratio of organic acid to pharmaceutical active varies from about 5:1 to 1:10 by weight.

Representative examples of water-insoluble polymers useful in the invention include ethylcellulose, polyvinyl acetate (for example, Kollicoat SR#30D from BASF), cellulose acetate, cellulose acetate butyrate, neutral copolymers based on ethyl acrylate and methylmethacrylate, copolymers of acrylic and methacrylic acid esters with quaternary ammonium groups such as Eudragit NE, RS and RS30D, RL or RL30D and the like. Representative examples of water-soluble polymers useful in the invention include polyvinylpyrrolidone (PVP), hydroxypropyl methylcellulose (HPMC), hydroxypropylcellulose (HPC), polyethylene glycol, and the like.

Representative examples of enteric polymers useful in the invention include esters of cellulose and its derivatives (cellulose acetate phthalate, hydroxypropyl methylcellulose phthalate, hydroxypropyl methylcellulose acetate succinate), polyvinyl acetate phthalate, pH-sensitive methacrylic acid-methamethacrylate copolymers and shellac. These polymers may be used as a dry powder or an aqueous dispersion. Some commercially available materials that may be used are methacrylic acid copolymers sold under the trademark Eudragit (L100, S100, L30D) manufactured by Rohm Pharma, Cellacefate (cellulose acetate phthalate) from Eastman Chemical Co., Aquateric (cellulose acetate phthalate aqueous dispersion) from FMC Corp. and Aqoat (hydroxypropyl methylcellulose acetate succinate aqueous dispersion) from Shin Etsu K.K.

The enteric, water-insoluble, and water-soluble polymers used in forming the membranes are usually plasticized. Representative examples of plasticizers that may be used to plasticize the membranes include triacetin, tributyl citrate, triethyl citrate, acetyl tri-n-butyl citrate diethyl phthalate, castor oil, dibutyl sebacate, acetylated monoglycerides and the like or mixtures thereof. The plasticizer, when used, may comprise about 3 to 30 wt.% and more typically about 10 to 25 wt.% based on the polymer. The type of plasticizer and its content depends on the polymer or polymers and nature of the coating system (e.g., aqueous or solvent based, solution or dispersion based and the total solids).

In general, it is desirable to prime the surface of the drug-layered particles before applying the barrier-membrane coatings or to separate the different membrane layers by applying a thin hydroxypropyl methylcellulose (HPMC) (e.g., Pharmacoat 603 or Opadry® Clear) film. While HPMC is typically used, other primers such as hydroxypropylcellulose (HPC) or lower viscosity ethylcellulose can also be used.

The active pharmaceutical ingredients suitable for incorporation into these time-controlled pulsatile release systems include weakly basic active pharmaceutical ingredients, derivatives, or salts thereof, exhibiting a pKa in the range of from about 5 to 14, a solubility of not more than about 200 µg/mL at pH 6.8 and a ratio of optimal highest dose to the solubility at pH 6.8 of at least about 100. The drug substance can be selected from the group of selective serotonin 5-HT₃ blocking agents having a pKa in the range of from about 5 to 14. A representative example is ondansetron or its hydrochloride salt with proven pharmacological activity in humans.

The membrane coatings can be applied to the core using any of the coating techniques commonly used in the pharmaceutical industry, but fluid bed coating is particularly useful. The present invention is directed to multi-dose forms, i.e., drug products in the form of multi-particulate dosage forms (hard gelatin capsules, conventional tablets or ODTs (orally disintegrating tablets)) comprising using a rotary tablet press one or more bead populations for oral administration to provide target PK profiles in patients in need of treatment. The conventional tablets rapidly disperse on entry into the stomach while ODTs rapidly disintegrate on contact with saliva in the oral cavity forming a smooth suspension of coated beads for easy swallowing. One or more coated bead populations may be compressed together with appropriate excipients into tablets (for example, a binder, a diluent/filler, and a disintegrant for conventional tablets while a rapidly dispersing granulation may replace the binder-diluent/filler combination in ODTs). Furthermore, compression into ODTs may be accomplished using a tablet press equipped with an external lubrication system to lubricate punches and dies prior to compression.

The following non-limiting examples illustrate the drug delivery dosage forms as capsules, conventional tablets or orally disintegrating tablets comprising one or more pulses, each with a predetermined delayed-onset and the totality of the *in vitro* drug-release profile or the ensuing in vivo plasma concentration profile upon oral administration of the dosage form should mimic the desired profile to achieve maximum therapeutic efficacy to enhance patient compliance and quality of life. Such dosage forms, when administered at the 'right time' or as recommended by the physician, would enable maintaining drug plasma concentration at a level potentially beneficial in minimizing the occurrence of side-effects associated with Cₘₐₓ or Cₘᵢₙ.

Example 1:

A. SR-Coated Fumaric Acid Crystals

40-80 mesh fumaric acid crystals (3750 g) were charged into a fluid-bed coater, Glatt GPCG 5 equipped with a 9" bottom spray Wurster insert, 10" column length and 16 mm tubing. These acid crystals were coated with a solution (at 6% solids) of 250 g of ethylcellulose (Ethocel Premium 10 cps) and 166.7 g of polyethylene glycol (PEG 400) at a ratio of 60/40 dissolved in 98/2 acetone/water (6528.3 g) for a weight gain of up to 10% by weight. The processing conditions were as follows: atomization air pressure: 2.0 bar, nozzle diameter: 1.00 mm; bottom distribution plate: B with 15 gauge 100 mesh screen; spray/shake interval: 30 s/3 s; product temperature maintained at 35±1°C; inlet air volume: 155-175 cubic feet per minute (cfm) and spray rate increased from about 8 to 30 g/min.

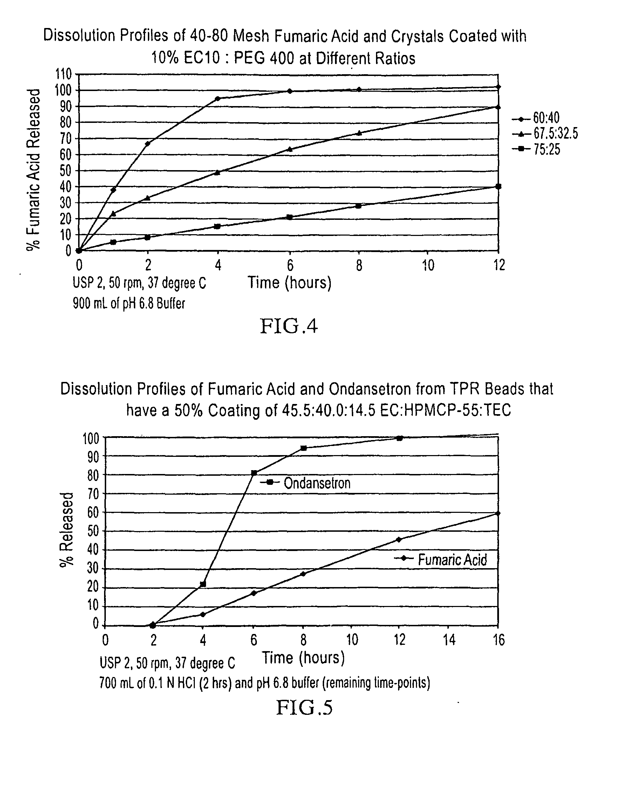

Fumaric acid crystals were also coated as described above using different ratios of ethylcellulose and PEG. More specifically, acid crystals were coated with a solution of EC-10 (Ethocel Premium 10 cps)/PEG 400 at a ratio of either 75/25 or 67.5/32.5 for a weight gain of up to 10% by weight in each case. Fig. 4 shows the fumaric acid release profiles from SR coated fumaric acid crystals coated at different ratios of EC-10/PEG.

B. Ondansetron Hydrochloride IR Beads Comprising SR-Coated Fumaric Acid Crystals

Povidone (PVP K-29/32; 23 g) was slowly added to 50/50 water/Denatured Alcohol 3C, 190 Proof (3699.4 g) while mixing to dissolve. Ondansetron hydrochloride dihydrate (197.2 g) was slowly added to the binder solution to dissolve the drug. SR-coated fumaric acid crystals (3000 g) obtained from above were coated in the Glatt GPCG 5 with the drug solution (5% solids) while maintaining the product temperature at 40±1°C; and inlet air volume at 180-195 cfm and spray rate being increased from about 8 to 15 g/min. The drug-layered beads were provided with a protective seal-coat of Opadry Clear (hypromellose 2910; 3 cps) (2% weight gain) to form IR beads.

C. Ondansetron Hydrochloride TPR Beads Comprising SR-Coated Fumaric Acid Crystals

Ondansetron hydrochloride IR beads (2800 g) from above were coated by spraying a solution in 98/2 acetone/water (6% solids) of EC-10/HPMCP (HP-55)/TEC (triethyl citrate) at a ratio of 45.5/40/14.5 for a weight gain of up to 50% and dried in the Glatt for about 10 minutes at 60°C to drive off excess residual solvent. The dried beads were sieved to discard any doubles formed.

Fig. 5 shows the release profiles of both fumaric acid and ondansetron from TPR beads comprising SR-coated acid crystals. More specifically, the TPR beads shown in Fig. 5 comprise IR beads (6% drug layered from 90/10 ondansetron/PVP) comprising fumaric acid crystals coated with EC-10/PEG 400 at a ratio of 67.5/32.5 at10% coated with EC-10/HP-55/TEC at a ratio of 45.5/40/14.5 for a weight gain of 50% by weight. Although the drug release is significantly faster than the acid release, it is apparent to a person skilled in the art that by decreasing the thickness of the barrier-coat (SR-coat) on the fumaric acid crystals and additionally applying a barrier-coat (SR-coat) under the TPR-coat to sustain the drug release, the release profiles for both ondansetron and fumaric acid can be synchronized.

**Example 2:**

In order to assess the type of *in vitro* release profile needed to achieve a once-daily plasma concentration profile, a modeling exercise was performed using the pharmacokinetic parameters for ondansetron hydrochloride reported in "Ondansetron Absorption in Adults: Effect of Dosage Form, Food, and Antacids" in Journal of Pharmaceutical Sciences Vol. (1994) by Bozigian et al.. Mean plasma concentrations achieved in 24 healthy, adult male volunteers, who received a single 8 mg ondansetron hydrochloride IR tablet in the fasted state, were used using the software program, WinNonlin^{™} Standard Version 2.1 to fit a 1-compartment first order model with a lag-time assuming first order elimination kinetics. The following parameters were obtained:

Primary Parameter: F = 1.0 (assumed); V_{d} = 238.26; Kₐ =1.49 per hour (hr); Ke = 0.19 per hr (hence t _{1/2} = 3.65 hr); T_{lag} = 0.41 hr. Secondary Parameters: AUC = 0.17 mg.hr/L; Cl = 46.06 L./hr; Tmax = 1.98 hrs; Cm = 0.0248 mg/L. These parameters very closely match the values reported in the above reference as well as in PDR.

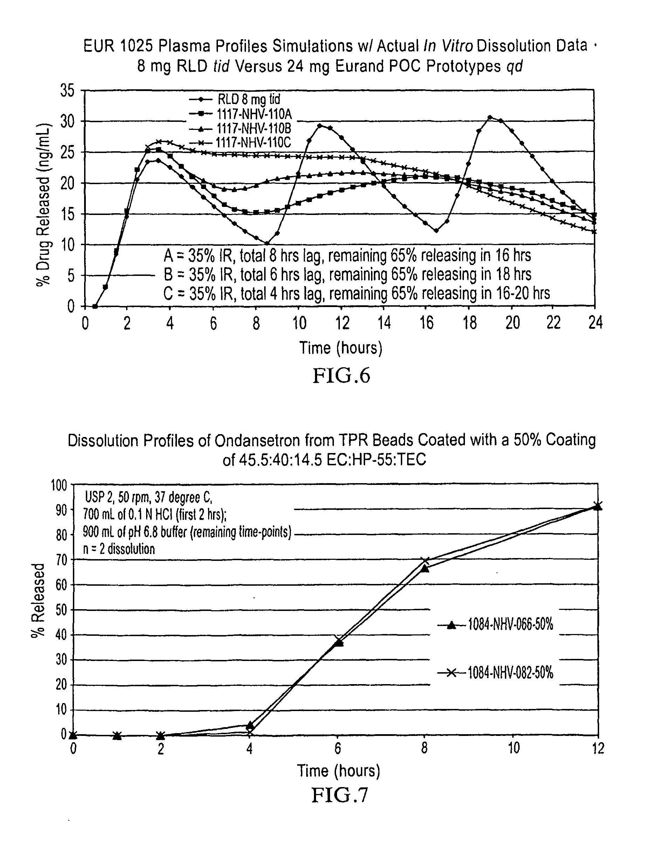

The primary parameters were then input into another software, Stella Version 6.01 using a previously established model with slight modifications. Different *in vitro* release profiles were generated, and from target once-daily release profiles, desired *in vitro* release (medium, target and fast) profiles were generated by deconvolution. These simulated plasma profiles are shown in Fig. 6.

**Example 3:**

A. Fumaric Acid-Containing Cores

Hydroxypropyl cellulose (Klucel LF, 23.9 g) was slowly added to denatured SD 3C 190 proof alcohol at 4% solids while stirring rigorously to dissolve and then fumaric acid (215.4 g) was slowly added to dissolve. Glatt GPCG 5 equipped with a 9" bottom spray Wurster insert, 10" partition column and 16 mm tubing was charged with 3750 g of 25-30 mesh sugar spheres. The sugar spheres were layered with the fumaric acid solution while maintaining the product temperature at about 33-34°C and inlet air velocity at flap opening of 38%. The acid cores were dried in the unit for 10 min to drive off residual solvent/moisture and sieved through 20-30 mesh screens.

B. SR-coated Fumaric acid Cores

The fumaric acid cores (3750 g) from above were coated with a solution of EC-10 and PEG 400 dissolved in 98/2 acetone/water (6% solids) for a weight gain of 10% by weight at two ratios, viz., (B.1) 60/40 and (B.2) 75/25 to examine its effect on the drug release from SR and TPR beads.

C. Ondansetron Hydrochloride IR Beads Comprising SR-coated Acid Cores

Povidone (PVP K-29/32, 19.5 g) was slowly added to 50/50 water/Denatured Alcohol 3C, 190 Proof (3699.4 g) while mixing to dissolve. Ondansetron hydrochloride dihydrate (175.2 g) was slowly added to the binder solution to dissolve the drug. SR-coated acid cores (3700 g) obtained from B.1 and B.2 above were coated in the Glatt GPCG 5 with the drug solution (5% solids).

D. Ondansetron Hydrochloride SR Beads

Ondansetron hydrochloride IR beads (3700 g) from above were barrier-coated (SR coated) by spraying a solution (7.5% solids) of 90/10 EC-10/TEC (triethyl citrate) at 5 and 10% by weight and dried in the Glatt for 10 minutes to drive off excess residual solvent. The dried beads were sieved to discard any doubles if formed.

E. Ondansetron Hydrochloride TPR Beads

Ondansetron hydrochloride SR beads (3500 g) from Example 3D were further coated with a lag-time coating membrane of EC-10/HP-55/TEC (triethyl citrate) at a ratio of 45.5/40.0/14.5 for a weight gain of about 30%, 40% and 50%. The TPR beads were dried in the Glatt at the same temperature to drive off residual solvent and sieved.

Fig. 7 shows the drug-release profiles of ondansetron hydrochloride from TPR beads (batch# 1084-066) comprising fumaric acid-containing cores coated with 60/40 EC-10/PEG 400 and TPR beads (batch# 1084-082) comprising fumaric acid-containing cores coated with 75/25 EC-10/PEG 400).

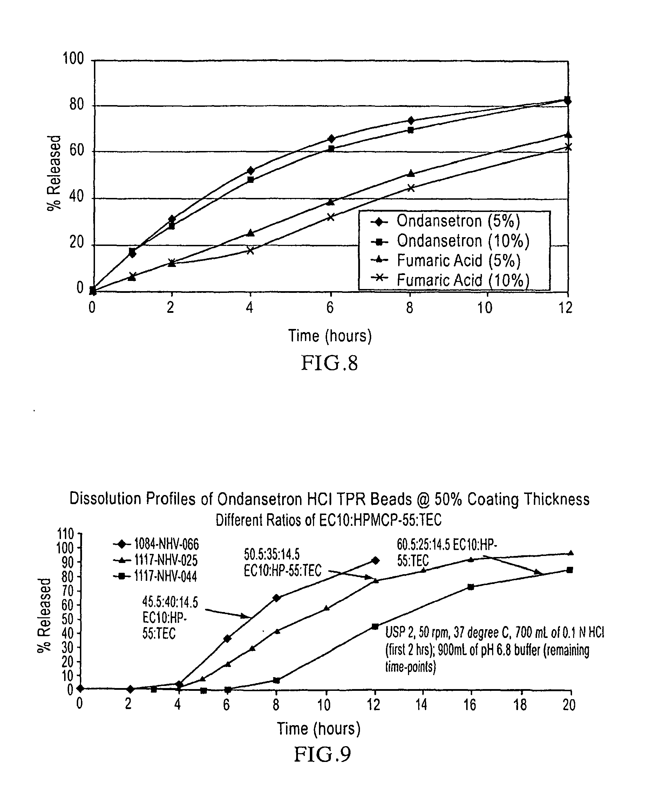

Fig. 8 shows the synchronized release profiles achieved for fumaric acid and ondansetron from SR beads (lot# 1084-060 - IR beads coated with 60/40 EC-10/PEG 400 at 5 and 10% by weight on fumaric acid-containing cores coated with 75/25 EC-10/PEG 400 at 10%).

**Example 4:**

A. Fumaric Acid-Containing Cores

Fumaric acid-containing cores were prepared by the procedure described in Example 3A excepting that 90/10 Denatured Alcohol (SD 3C, 190 Proof)/water was used instead of the alcohol alone.

B. SR-coated Fumaric Acid-Containing Cores

The fumaric acid cores (3750 g) from above were coated with a solution of EC-10 and either PEG 400 (B.1) at a ratio of 60/40 or TEC (B.2) at a ratio of 90/10 as the plasticizer, dissolved in 98/2 acetone/water (6% solids) for a weight gain of 10%.

C. Ondansetron Hydrochloride IR Beads

Ondansetron hydrochloride IR beads from B.1 and B.2 above were prepared as disclosed in Example 3 C. The drug-layered beads were provided with a protective seal-coat with Pharmacoat 603 (hypromellose 2910; 3 cps) for a weight gain of 2%.

D. Ondansetron Hydrochloride SR Beads

Ondansetron hydrochloride IR beads (1080 g) were barrier-coated (SR coated) by spraying a solution of EC-10 and either PEG 400 (D.1) at a ratio of 60/40 or TEC (D.2) at a ratio of 90/10 as the plasticizer, dissolved in 98/2 acetone/water (7.5% solids) for a weight gain of 10% and dried in the Glatt at the same temperature for 10 minutes to drive off excess residual solvent. The dried beads were sieved to discard any doubles if formed.

E. Ondansetron Hydrochloride TPR Beads

Ondansetron hydrochloride SR beads from D.1 and D.2 above were further coated with a lag-time coating membrane of EC-10/HP-55/TEC at three ratios of 45.5/40/14.5 (E.1 - lot# 1084-066), 50.5/35/14.5 (E.2 - lot# 1117-025) and 60.5/25/14.5 (E.3 - lot# 1117-044) dissolved in 90/10 acetone/water (7.5% solids) for a gain of up to 50% by weight. The TPR beads were dried in the Glatt to drive off residual solvent and sieved through a 18 mesh sieve. Fig. 9 shows the release profiles for ondansetron hydrochloride from TPR beads coated with EC-10/HP-55/TEC at three different ratios (E.1, E.2 and E.3). More specifically, Fig. 9 shows the release profiles for the following formulations:
(1) TPR beads lot# 1084-066-The coating of EC-10/HP-55/TEC at a ratio of 45.5/40/14.5 at 50% by weight applied on IR beads coated with 60/40 EC-10/PEG 400 at 10% while IR beads (5% drug layered from 90/10 ondansetron/PVP) comprise fumaric acid cores (4% layered on sugar spheres from acid/Klucel) coated with 60/40 EC-10/PEG 400 at 10%.
(2) TPR beads lot# 1117-025 - The coating of EC-10/HP-55/TEC at a ratio of 50.5/35/14.5 at 50% by weight applied on IR beads coated with 90/10 EC-10/TEC at 10% while IR beads (6% drug layered from 90/10 ondansetron/ Klucel LF) comprise fumaric acid cores (layered on sugar spheres from acid/PVP) coated with 90/10 EC-10/TEC at 10%.
(3) TPR beads lot# 1117-044- The coating of EC-10/HP-55/TEC at a ratio of 60.5/25/14.5 at 50% by weight applied on IR beads coated with 90/10 EC-10/TEC at 10% while IR beads (6% drug layered from 90/10 ondansetron/Klucel LF) comprise fumaric acid cores (layered on sugar spheres from acid/PVP) coated with 90/10 EC-10/TEC at 10%.

**Example 5:**

### A. Fumaric Acid-Containing Cores

Fumaric acid-containing cores were prepared by the procedure described in Example 3A excepting that fumaric content in thc acid-containing cores was 11.25% instead of 6% in Example 3A,

### B. SR-caated humaric Acid-Containing Cores

The fumaric acid-containing cores (3750 g) from above were coated with a solution of EC-10/TEC at a ratio of 90/10 dissolved in 95/5 acetone/water (7.5% solids) for a weight gain of 5%.

### C. Ondansetron Hydrochloride IR Beads

Ondansetron hydrochloride IR beads from above were prepared as disclosed in Example 3 C.

### D. Ondansetron Hydrochloride SR Beads

Ondansetron hydrochloride IR beads (3500 g) were barrier-coated by spraying a solution (7.5% solids) of 90/10 EC-10/TEC dissolved in 95/5 acetone/water at 10% by weight and dried in the Glatt for 10 minutes to drive off excess residual solvent. The dried beads are sieved through a 18 mesh sieve to discard any doubles if formed.

### E. Ondansetron Hydrochloride TPR Beads

Ondansetron hydrochloride SR beads (2600 g) from above were further coated with a lag-time coating membrane of EC-10/HP-55/TEC at a ratio of 60.5/25/14.5 dissolved in 90/10 acetone/water (7.5% solids) for a weight gain of 30%, 45%, and 50%. The coated beads were cured at 60°C for 30 minutes in the same unit and sieved through a 18 mesh sieve after cooling to ambient temperature.

F. Ondansetron Hydrochloride MR Capsules

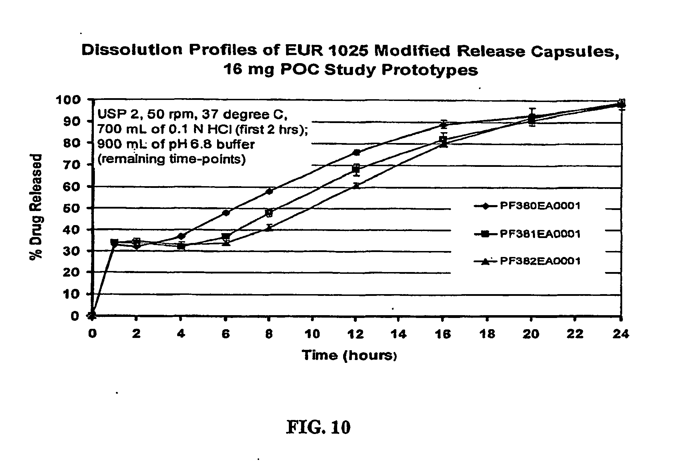

Ondansetron hydrochloride IR beads (PE364EA0001) and TPR beads (lot# PE366EA0001 with a lag-time coating of 30%, lot# PE367EA0001 with a lag-time coating of 45%, and lot# PE368EA0001 with a lag-time coating of 50%) were encapsulated at a ratio of 35% / 65% into hard gelatin capsules to produce MR (modified-release) Capsules, 16 mg (lots# PF380EA0001, lots# PF381EA0001, and lots# PF382EA0001) QD (dosed once-daily) for a pilot bioavailability study in humans in comparison to marketed Zofran® 8 mg (as ondansetron) dosed bid (two times a day). Fig. 10 shows the drug-release profiles from the three MR Capsules comprising IR and TPR beads.

**Example 6:**

A. Fumaric Acid-Containing Cores

60-80 mesh sugar spheres (933.3 g) would be layered with fumaric acid (240 g) from a solution (4% solids) of Klucel LF (26.7 g) as disclosed in Example 3 to achieve an acid load of 20% by weight. The acid cores are dried in the unit for 10 min to drive off residual solvent/moisture and sieved through 40-80 mesh screens.

B. SR-coated Fumaric acid Cores

The acid cores (910 g) from above are coated with a solution of 441.5 g of ethylcellulose (EC-10) and 49 g of triethyl citrate (TEC) at a ratio of 90/10 dissolved in 95/5 acetone/water (7.5% solids) for a weight gain of 35%.

C. Ondansetron Hydrochloride IR Beads

IR beads of ondansetron hydrochloride dihydrate with a drug load of 11.13% by weight would be produced following the procedures disclosed in Example 5C. Ondansetron hydrochloride dihydrate (140.4 g) and Klucel LF (15.6 g) solution would be layered onto SR-coated acid-containing cores (1080 g) and a seal-coat of Pharmacoat 603 would be applied for a weight gain of 2%.

D. Ondansetron Hydrochloride SR Beads

Ondansetron hydrochloride IR beads 1080 g would be barrier-coated (SR coated) by spraying a solution (7.5% solids) of 90/10 EC-10/TEC at 5 and 10% by weight and dried in the Glatt at the same temperature for 10 minutes to drive off excess residual solvent. The dried beads are sieved to discard any doubles if formed.

E. Ondansetron Hydrochloride TPR Beads

Ondansetron hydrochloride SR beads would be further coated with a lag-time coating membrane of EC-10/HP-55/TEC at a ratio of 60.5/25/14.5 for a weight gain of 30%, 35% and 40%. The TPR beads would be cured at 60°C for 30 minutes in the Glatt to drive off residual solvent and sieved through 30 mesh sieve.

F. Rapidly-dispersible microgranules

The rapidly-dispersible microgranules comprising a sugar alcohol such as mannitol and a disintegrant such as crospovidone would be prepared following the procedure disclosed in the co-pending US Patent Application Publication No. U.S. 2005/0232988, published October 20, 2005, the contents of which are hereby incorporated by reference. D-mannitol (152 kg) with an average particle size of approximately 20µm or less (Pearlitol 25 from Roquette, France) is blended with 8 kg of cross-linked povidone (Crospovidone XL-10 from ISP) in a high shear granulator (GMX 600 from Vector) and granulated with purified water (approximately 32 kg) and wet-milled using Comil from Quadro and dried in Glatt GPCG 200. The rapidly-dispersible microgranules thus obtained would have an average particle size in the range of approximately 125-200µm.

G. Ondansetron Hydrochloride MR ODT, 12 mg:

Rapidly-dispersible microgranules (2541.2 g) would be blended with TPR beads (460.8 g), SR beads (479.0 g), IR beads (377.4 g) and other pharmaceutical acceptable ingredients (142.0 g), such as flavor, sweetener, and additional disintegrant, in a twin shell V-blender for a sufficient time to get homogeneously distributed blending for compression. Tablets weighing approximately 400 mg would be compressed using a production scale tablet press equipped with an external lubrication system at a mean hardness of about 4-5 kP. Ondansetron Hydrochloride Dihydrate MR ODT, 12 mg thus produced would rapidly disintegrate in the oral cavity creating a smooth, easy-to-swallow suspension comprising coated ondansetron hydrochloride beads, which would provide a target profile suitable for a once-daily dosing regimen.

**Example 7:**

A 4-arm crossover pilot POC (proof of concept) study was conducted which included 12 Caucasian male, healthy volunteers aged 18 to 55 years with a wash-out period of 7 days. Each volunteer was dosed with 250 mL of mineral water a single dose of 16 mg Test Formulation (either A (PF380EA0001), B (PF381EA0001), or C (PF382EA0001) of Example 4) at 8 AM or two 8 mg Zofran® (i.e., one at 8 AM and the other at 4:30 PM after an overnight fasting (at least 12 hrs), and lunch was served at 11 AM. Blood samples were drawn at 0 (pre-dose), 20 min, 40 min, 1 hr, 1.5 hrs, 2 hrs,, 3 hrs, 4 hrs, 6 hrs, 8.5 hrs (before second dose), 9 hrs 10 min, 9.5 hrs, 10 hrs, 10.5s, 11.5 hrs, 12.5 hrs, 14.5 hrs, 17 hrs, 20 hrs, 22 hrs, 24 hrs and 36 hrs. The PK (pharmacokinetics) parameters are presented in Table 2. The table demonstrates that the plasma profiles of Test Formulations A (PE280EA0001), B (PE281EA0001), and C (PE282EA0001) are those characteristic of sustained release formulations, i.e., apparent half-life is significantly longer than that with Zofran. AUC or Cₘₐₓ of Test Formulations does not deviate substantially from that of Zofran (i.e., AUC within ±25% and Cₘₐₓ approximately 70% of Zofran). The actual Cₘₐₓ for Zofran 8 mg was 30 ng/mL in comparison to the predicted 24 ng/mL while the actual Cₘₐₓ for the IR component was about 24 ng/mL when normalized. Approximately 70% of Zofran 8 mg bid (twice-dosed) was absorbed in 24 hrs. Test Formulations A to C exhibited the expected trend post-dosing up to the crossover point at about 15-16 hrs; thereafter, Formula C continued to exhibit a lower plasma concentration-time profile contrary to the predicted behavior.

From these demonstrations, it is apparent that the incorporation of an organic acid, as the solubilizer for the weakly basic drugs exhibiting a pH-dependent solubility profile (i.e., showing a decrease in solubility at the intestinal pH 6.8 by about 2 orders of magnitude in comparison to its maximum solubility in the GI fluid) and functional coating of the acid before applying the active pharmaceutical ingredient has significant impact on the lag time, a desired but complete drug release profile prior to depletion of the buffer. Furthermore, the active pharmaceutical ingredient remains in the unaltered form in the solid dosage form until it is released for absorption in the GI tract.

**Table 2: PK Parameters of Example 7**

| **Formula A** | **Cₘₐₓ** | **Tₘₐₓ** | **AUCₗₐₛₜ** | **AUC_{inf}** | **t_{1/2}** |
|---|---|---|---|---|---|
| **Mean** | 19.452 | 4.8055 | 358.71 | 424.21 | 11.677 |
| SD | 4,1207 | 4.2174 | 125.28 | 162.14 | 2.3797 |
| Median | 19.193 | 2.5 | 353.56 | 404.36 | 10.993 |
| Minimum | 11.475 | 1.5 | 160.09 | 200.93 | 7.9295 |
| Maximum | 25.327 | 12.5 | 583.2 | 747.75 | 15.53 |
| | | | | | |

| **Formula B** | **Cₘₐₓ** | **Tₘₐₓ** | **AUCₗₐₛₜ** | **AUC_{inf}** | **t_{1/2}** |
|---|---|---|---|---|---|
| **Mean** | 20.754 | 1.9583 | 341.61 | 445.28 | 15.338 |
| SD | 3.6564 | 0.8107 | 78.421 | 106.68 | 7.4115 |
| Median | 21.116 | 1.75 | 336.09 | 473.84 | 13.658 |
| Minimum | 12.699 | 1 | 226.66 | 236.61 | 5.745 |
| Maximum | 27.137 | 4 | 482.75 | 582.18 | 32.606 |
| | | | | | |

| **Formula C** | **Cₘₐₓ** | **Tₘₐₓ** | **AUCₗₐₛₜ** | **AUC_{inf}** | **t_{1/2}** |
|---|---|---|---|---|---|
| **Mean** | 19.73 | 2.9167 | 313.83 | 391.35 | 13.995 |
| SD | 5.3751 | 2.0207 | 71.218 | 92.355 | 4.9522 |
| Median | 20.062 | 2.5 | 315.51 | 388.6 | 13.255 |
| Minimum | 11.022 | 1 | 195.87 | 240.77 | 6.1444 |
| Maximum | 27.299 | 8.5 | 401.82 | 519.33 | 22.231 |
| | | | | | |

| **Zofran** | **Cₘₐₓ** | **Tₘₐₓ** | **AUCₗₐₛₜ** | **AUC_{inf}** | **t_{1/2}** |
|---|---|---|---|---|---|
| **Mean** | 38.471 | 8.0833 | 460.81 | 487.17 | 7.10004 |
| SD | 9.5092 | 4.1661 | 124.18 | 144.94 | 2.4726 |
| Median | 35.655 | 9.75 | 460.52 | 475.48 | 6.945 |
| Minimum | 27.37 | 1 | 309.94 | 320.19 | 3.5092 |
| Maximum | 54.502 | 12.5 | 687.39 | 788.77 | 11.815 |

The following numbered paragraphs contain statements of broad combinations of the inventive technical features herein disclosed:-
1. A pharmaceutical multiparticulate dosage form comprising immediate release (IR) beads, sustained-release (SR) beads and/or one or more populations of timed, pulsatile release (TPR) beads of a weakly basic drug, wherein the weakly basic drug comprises a nitrogen (N)-containing selective serotonin 5-HT₃ blocking agent having a pKa in the range of from about 5 to 14 and a solubility of not more than about 200 µg/mL at pH 6.8 and at least one pharmaceutically acceptable organic acid as a solubilizer wherein the weakly basic active and the organic acid do not come into contact with each other during manufacturing or in storage in the solid state thereby avoiding *in-situ* formation of an acid addition compound and the organic acid is not depleted until completion of the drug release from the dosage form when dissolution tested by United States Pharmacopoeia (USP) dissolution methodology using a two-stage dissolution medium (first 2 hours in 0.1N HCl followed by testing in a buffer at pH 6.8).
2. A pharmaceutical multiparticulate dosage form in accordance with para 1 wherein the ratio of optimal highest dose for the weakly basic drug to solubility at pH 6.8 is not less than about 100, and at least one pharmaceutically acceptable organic acid solubilizes said weakly basic drug prior to releasing it into a hostile intestinal environment wherein said weakly basic drug is practically insoluble, and said dosage form exhibits target pharmacokinetics profiles at 24 hour post-dosing suitable for a once-daily dosing regimen in patients in need of such a medication.
3. A pharmaceutical multiparticulate dosage form in accordance with para 1 wherein:
   a) said TPR bead comprises an outer lag-time coating comprising a water-insoluble polymer in combination with an enteric polymer applied over said SR bead, said outer coating providing a lag time of from about 2 to about 7 hours before onset of drug release;
   b) said SR bead comprises an SR (barrier) coating surrounding an IR bead, said SR coating comprising a water-insoluble polymer alone or in combination with a water-soluble pore-forming polymer, said SR coating providing a sustained-release profile;
   c) said IR bead comprises at least one weakly basic drug applied on an SR-coated organic acid core particle;
   d) said SR-coated organic acid core comprises an inner barrier coating surrounding an organic acid core particle, said inner barrier coating comprising a water-insoluble polymer alone or in combination with a water-soluble pore-forming polymer and providing a sustained-release profile; and
   e) said organic acid core particle comprises at least one pharmaceutically acceptable organic acid functioning as a solubilizer of said weakly basic drug.
4. A pharmaceutical multiparticulate dosage form in accordance with para 1 in the form of an orally disintegrating tablet (ODT) further comprising:
   1) rapidly-dissolving microgranules with an average particle size of not more than 400 µm comprising a disintegrant and a sugar alcohol or a saccharide or a combination thereof, each having an average particle size of not more than about 30 µm;
   wherein said orally disintegrating tablet disintegrates into multi-coated beads on contact with the saliva in the oral cavity within approximately 60 seconds or less.
5. A pharmaceutical multiparticulate dosage form in accordance with para 3 wherein said TPR beads do not include a barrier (SR) coating on said IR beads thereby enabling the release of solubilized drug into a hostile intestinal environment wherein the drug is practically insoluble following oral administration in order to be suitable for a once-daily dosing regimen in patients in need of such a medication.
6. A pharmaceutical multiparticulate dosage form in accordance with para 1 comprising at least an IR bead population, a first TPR bead population, and an SR bead population or a second TPR bead population and a wherein the ratio of IR bead population to the first TPR bead population to the SR bead or second TPR bead populations varies from about 10:90:0 to about 40:10:50.
7. A pharmaceutical multiparticulate dosage form in accordance with para 1 wherein said weakly basic nitrogen (N)-containing selective serotonin 5-HT₃ blocking agent is ondansetron or a pharmaceutically acceptable salt thereof having a pKa of 7.4 and a solubility of less than 100 µg/mL at a pH of 6.8, a selective 5-HT₃ receptor antagonist indicated for the prevention of nausea and vomiting associated with chemotherapy or post-operative surgery.
8. A pharmaceutical multiparticulate dosage form in accordance with para 1 wherein the organic acid is selected from the group consisting of citric acid, fumaric acid, malic acid, maleic acid, tartaric acid, succinic acid, oxalic acid, aspartic acid, glutamic acid and mixtures thereof.
9. A pharmaceutical multiparticulate dosage form in accordance with para 1 wherein the ratio of weakly basic drug to organic acid varies from about 5:1 to 1:10 by weight to provide target pharmacokinetic profiles suitable for a once-daily dosing regimen.
10. A pharmaceutical multiparticulate dosage form in accordance with para 3, wherein said organic acid core particle comprises:
   i) an organic acid crystal;
   ii) an inert particle coated with an organic acid and a polymeric binder; or
   iii) a pellet or a micro-tablet containing the organic acid, a polymeric binder and a diluent/filler, prepared by rotogranulation, granulation-extrusion-spheronization or granulation-compression.
11. A pharmaceutical multiparticulate dosage form in accordance with para 3 wherein said particle core is provided with a barrier (SR) coating comprising a water-insoluble polymer alone or in combination with a water soluble polymer at a ratio of from about 9:1 to 5:5 wherein said barrier coating is applied for a weight gain of from about 1.5% to 20% by weight based on the weight of the coated bead.
12. A pharmaceutical multiparticulate dosage form in accordance with para 11
   wherein said particle barrier coating comprises a water-insoluble polymer selected from the group consisting of ethylcellulose, cellulose acetate, cellulose acetate butyrate, polyvinyl acetate, neutral methacrylic acid-methylmethacrylate copolymers, and mixtures thereof.
13. A pharmaceutical multiparticulate dosage form in accordance with para 11
   wherein said particle core is provided with a barrier coating comprising a water-insoluble polymer in combination with a water soluble polymer selected from the group consisting of methylcellulose, hydroxypropyl methylcellulose, hydroxypropylcellulose, polyvinyl pyrrolidone and polyethylene glycol and mixtures thereof.
14. A pharmaceutical multiparticulate dosage form in accordance with para 3 wherein said lag-time coating comprises a water-insoluble polymer in combination with an enteric polymer at a ratio of from about 9:1 to 1:3, respectively, for a weight gain of from about 10% to 60% by weight based on the weight of the TPR bead.
15. A pharmaceutical multiparticulate dosage form in accordance with para 14
   wherein said lag-time coating comprises a water-insoluble polymer in combination with an enteric polymer selected from the group consisting of cellulose acetate phthalate, hydroxypropyl methylcellulose phthalate, hydroxypropyl methylcellulose succinate, polyvinyl acetate phthalate, pH-sensitive methacrylic acid-methylmethacrylate copolymers, shellac, derivatives thereof, and mixtures thereof.
16. A pharmaceutical multiparticulate dosage form in accordance with para 3 wherein at least one of the inner barrier coatings and the outer lag-time coating comprises a plasticizer selected from the group consisting of triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides and mixtures thereof.
17. A pharmaceutical multiparticulate dosage form of para 3 wherein said IR beads provide a loading dose by releasing not less than about 50% of the active contained in said IR beads within the first hour after oral administration of the dosage form.
18. A pharmaceutical multiparticulate dosage form of para 3 wherein said IR bead, if incorporated as an IR portion of the dosage form, comprises said weakly basic drug and a polymer binder layered on an inert core.
19. A pharmaceutical multiparticulate dosage form in accordance with para 1 wherein said weakly basic drug comprises ondansetron or pharmaceutically acceptable salt thereof and each TPR bead population comprises sustained-release coated fumaric acid cores coated with a lag-time coating of water-insoluble ethylcellulose and enteric hydroxypropyl methylcellulose phthalate at a ratio of from about 9:1 to about 1:3 for a weight gain of up to 50%, exhibiting upon oral administration of the dosage form a pre-determined lag-time followed by differing release characteristics.
20. A pharmaceutical multiparticulate dosage form in accordance with para 1 wherein said orally disintegrating tablet comprises a taste-masked IR bead population, an SR bead population, and /or one or two TPR bead populations of ondansetron hydrochloride dihydrate, wherein each SR or TPR bead population comprising sustained-release coated fumaric acid cores, rapidly disintegrates in the oral cavity creating a smooth, easy-to-swallow suspension of multi-coated beads to provide target pharmacokinetics profiles suitable for a once-daily dosing regimen in patients in need of such a medication.
21. A method for the preparation of a multiparticulate dosage form comprising a weakly basic, nitrogen (N)-containing selective serotonin 5-HT₃ blocking agent having a pKa in the range of from about 5 to 14, a solubility of not more than 200 µg/mL at a pH of 6.8, and a ratio of optimal highest dose to solubility at pH 6.8 of not less than about 100, and at least one pharmaceutically acceptable organic acid as a solubilizer comprising:
   a) preparing organic acid cores;
   b) preparing SR-coated organic acid cores by coating the organic acid cores with a SR coating comprising a water-insoluble polymer alone or in combination with a water-soluble polymer or an enteric polymer at a ratio of from about 95:5 to about 50:50 for a weight gain of up to about 20%, to provide a sustained-release profile;
   c) preparing IR (immediate-release) beads by layering the weakly basic or pharmaceutically acceptable salt thereof from a polymer binder solution onto the SR-coated organic acid cores and optionally applying a protective seal-coat with a water-soluble polymer;
   d) preparing SR beads by applying a barrier (SR) coating of a water-insoluble polymer alone or in combination with a water-soluble polymer at a ratio of from about 95:5 to about 50:50 for a weight gain of from about 1.5% to 20% by dry weight of the coated bead;
   e) preparing TPR beads by applying an outer lag-time coating comprising a water-insoluble polymer in combination with an enteric polymer at a ratio of from about 9:1 to 1:3 for a weight gain of from about 10% to 60% by weight of the coated bead; and
   f) filling into a gelatin capsule or compressing into a conventional tablet or an orally disintegrating tablet a mixture of IR beads, SR beads and/or one or more TPR bead populations at appropriate amounts to achieve target pharmacokinetics profiles in order to be suitable for a once-daily dosing regimen in patients in need of such a medication.
22. A method in accordance with para 21, wherein each of said organic acid-layering, SR-coating, drug-layering and outer lag-time coating is applied from a solution in a pharmaceutically acceptable solvent system or from an aqueous dispersion.
23. A method in accordance with para 21 further comprising:
   g) optionally taste-masking drug-containing beads either by solvent coacervation or by fluid-bed coating;
   h) granulating a sugar alcohol or a saccharide, or a combination thereof, and a disintegrant, each having an average particle size of not more than about 30 µm to produce rapidly dispersing microgranules with an average particle size of not more than about 400 µm;
   i) blending multi-coated beads with the rapidly dispersing microgranules at a ratio of multi-coated beads to microgranules from about 1:6 to about 1:2; and
   j) compressing the blend of step (i) into orally disintegrating tablets using a rotary tablet press.
24. A method in accordance with para 23, wherein said step of compressing into orally disintegrating tablets comprises utilizing a conventional rotary tablet press equipped with an external lubrication system to lubricate the dies and punches prior to compression.
25. A method of para 21 wherein the dosage form comprises therapeutically effective amounts of IR bead population, SR bead population and/or one or more TPR bead populations of a weakly basic, nitrogen (N)-containing selective serotonin 5-HT₃ blocking agent, each multicoated bead population exhibiting differing release characteristics following a pre-determined lag-time.

## Claims

1. A pharmaceutical multiparticulate dosage form comprising one or more populations of timed, pulsatile release (TPR) beads of a weakly basic drug;
wherein the weakly basic drug comprises a nitrogen (N)-containing selective serotonin 5-HT₃ blocking agent, or a pharmaceutically acceptable salt thereof, having a pKa in the range of from about 5 to 14 and a solubility of not more than about 200 µg/mL at pH 6.8;
wherein the TPR beads comprise organic acid core particles comprising at least one pharmaceutically acceptable organic acid, which are barrier coated with a SR (sustained release) polymer membrane comprising a water-insoluble polymer alone or in combination with a water-soluble polymer or an enteric polymer, on which the weakly basic drug is layered and further coated with a lag-time membrane such that both the organic acid and the weakly basic drug exhibit comparable drug-release profiles; and
wherein the weakly basic drug and the organic acid do not contact each other in the dosage form.

2. The pharmaceutical multiparticulate dosage form of claim 1, wherein the weakly basic drug has a ratio of optimal highest dose to solubility at pH 6.8 of not less than about 100, and at least one pharmaceutically acceptable organic acid solubilizes said weakly basic drug prior to releasing it into a hostile intestinal environment wherein said weakly basic drug is practically insoluble, and said dosage form exhibits a pharmacokinetic profile at 24 hours post-dosing suitable for a once-daily dosing regimen for patients in need thereof.

3. The pharmaceutical multiparticulate dosage form of claim 1, wherein said TPR beads comprise an outer lag-time coating comprising a water-insoluble polymer in combination with an enteric polymer, said outer lag-time coating providing a lag time of from about 2 to about 7 hours before onset of release of the weakly basic drug;
wherein said outer lag-time coating is disposed over a sustained-release (SR) coating comprising a water-insoluble polymer alone or in combination with a water soluble polymer, said SR coating providing a sustained release profile,
wherein said SR coating is disposed over the weakly basic drug layer; and wherein the weakly basic drug layer is disposed over the inner barrier coating, which is disposed over the organic acid containing cores, said inner barrier coating providing a sustained-release profile.

4. The pharmaceutical multiparticulate dosage form of claim 1, in the form of an orally disintegrating tablet (ODT), wherein, optionally,
(a) said ODT further comprises rapidly-dispersing microgranules which comprise a disintegrant and a sugar alcohol or a saccharide or a combination thereof, and each of the disintegrant and sugar alcohol or saccharide have an average particle size of not more than about 30 µm,
(b) said microgranules have an average particle size of not more than 400 µm, and
(c) said ODT optionally has a disintegration time of about 60 seconds or less on contact with saliva in the oral cavity.

5. The pharmaceutical multiparticulate dosage form of any one of claims 1 to 4,
wherein the weakly basic drug is ondansetron hydrochloride and the pharmaceutically acceptable organic acid is fumaric acid.

6. The pharmaceutical multiparticulate dosage form of any preceding claim, wherein said weakly basic drug is ondansetron, or a pharmaceutically acceptable salt thereof having a pKa of 7.4 and a solubility of less than 100 µg/mL at a pH of 6.8.

7. The pharmaceutical multiparticulate dosage form of claim 1, wherein
(a) the organic acid is selected from the group consisting of citric acid, fumaric acid, malic acid, maleic acid, tartaric acid, succinic acid, oxalic acid, aspartic acid, glutamic acid, and mixtures thereof;
(b) the ratio of the weakly basic drug to the organic acid varies from about 5:1 to 1:10 by weight, to provide target pharmacokinetic profiles suitable for a once-daily dosing regimen; or
(c) the organic acid is not depleted from said dosage form until completion of the release of the weakly basic drug from the dosage form when dissolution tested by United States Pharmacopoeia (USP) dissolution methodology using a two-stage dissolution medium (first 2 hours in 0.1N HCl followed by testing in a buffer at pH 6.8).

8. The pharmaceutical multiparticulate dosage form of claim 1 or claim 3, wherein said organic acid core particles comprise:
i) an organic acid crystal;
ii) an inert particle coated with an organic acid and a polymeric binder; or
iii) a pellet or a micro-tablet comprising the organic acid, a polymeric binder and a diluent/filler, prepared by rotogranulation, granulation-extrusion-spheronization or granulation-compression.

9. The pharmaceutical multiparticulate dosage form of claim 3, wherein
(a) the SR coating comprises a water-insoluble polymer alone, or a water-insoluble polymer in combination with a water soluble polymer at a ratio of from about 9:1 to 5:5, and wherein said SR coating is applied for a weight gain of from about 1.5% to 20% by weight based on the total weight of the SR-coated organic acid core particles
(b) said lag-time coating comprises a water-insoluble polymer in combination with an enteric polymer at a ratio of from about 9:1 to 1:3, respectively, for a weight gain of from about 10% to 60% by weight based on the dry weight of the TPR beads,
(c) the lag-time coating comprises ethyl cellulose with an average viscosity of 10 cps and hydroxypropylmethyl cellulose phthalate and optionally further comprises a plasticizer, or
(d) the lag-time coating comprises ethyl cellulose with an average viscosity of 10 cps and hydroxypropylmethyl cellulose phthalate and the weakly basic drug is ondansetron or a pharmaceutically acceptable salt thereof having a pKa of 7.4 and a solubility of less than 100 µg/mL at a pH of 6.8.

10. The pharmaceutical multiparticulate dosage form of claim 1, wherein
(a) the water-insoluble polymer is selected from the group consisting of ethylcellulose, cellulose acetate, cellulose acetate butyrate, polyvinyl acetate, neutral methacrylic acid-methylmethacrylate copolymers, and mixtures thereof
(b) the water soluble polymer is selected from the group consisting of methylcellulose, hydroxypropyl methylcellulose, hydroxypropylcellulose, polyvinyl pyrrolidone and polyethylene glycol, and mixtures thereof
(c) the enteric polymer is selected from the group consisting of cellulose acetate phthalate, hydroxypropyl methylcellulose phthalate, hydroxypropyl methylcellulose succinate, polyvinyl acetate phthalate, pH-sensitive methacrylic acid-methylmethacrylate copolymers, shellac, derivatives thereof, and mixtures thereof, and/or
(d) at least one of the SR coating, the inner barrier coating and the outer lag-time coating further comprises a plasticizer selected from the group consisting of triacetin, tributyl citrate, tri-ethyl citrate, acetyl tri-n-butyl citrate, diethyl phthalate, dibutyl sebacate, polyethylene glycol, polypropylene glycol, castor oil, acetylated mono- and di-glycerides, and mixtures thereof.

11. The pharmaceutical multiparticulate dosage form of claim 1 or claim 3, wherein said weakly basic drug comprises ondansetron or a pharmaceutically acceptable salt thereof; each TPR bead population comprises SR-coated organic acid core particles comprising fumaric acid; and the lag-time coating comprises ethylcellulose and hydroxypropyl methylcellulose phthalate at a ratio of from about 9:1 to about 1:3, for a weight gain of 5% to 60%, each population of TPR beads exhibiting upon oral administration of the dosage form a pre-determined lag-time and differing release characteristics.

12. A method for the preparation of the pharmaceutical multiparticulate dosage form comprising one or more populations of timed, pulsatile release (TPR) beads of a weakly basic nitrogen (N)-containing selective serotonin 5-HT₃ blocking agent, or a pharmaceutically acceptable salt thereof, having a pKa in the range of from about 5 to 14 and a solubility of not more than about 200 µg/mL at pH 6.8 and a ratio of optimal highest dose to solubility at pH 6.8 of not less than about 100, and at least one pharmaceutically acceptable organic acid as a solubiliser comprising:
a) preparing organic acid core particles comprising at least one pharmaceutically acceptable organic acid;
b) preparing SR-coated organic acid core particles by coating the organic acid core particles with aSR coating comprising a water-insoluble polymer alone, or a water-insoluble polymer in combination with a water-soluble polymer or an enteric polymer at a ratio of from about 95:5 to about 50:50 for a weight gain of up to about 20%, to provide a sustained release profile;
c) preparing IR (immediate-release) beads by coating a solution comprising the weakly basic drug, or a pharmaceutically acceptable salt thereof, and a polymer binder, and optionally applying a protective seal-coat comprising a water-soluble polymer, onto the inner barrier-coated organic acid core particles;
d) optionally preparing SR beads by applying an SR (barrier) coating comprising a water-insoluble polymer alone, or a water -insoluble polymer in combination with a water-soluble polymer at a ratio of from about 95:5 to about 50:50 onto the IR beads, for a weight gain of from about 1.5% to 20% of the total dry weight of the coated SR beads;
e) preparing TPR beads by applying an outer lag-time coating comprising a water-insoluble polymer in combination with an enteric polymer to the beads of step (c) or (d), at a ratio of from about 9:1 to 1:3 for a weight gain of from about 10% to 60% of the total dry weight of the TPR beads; and
f) filling into a capsule or compressing into a tablet or an orally disintegrating tablet, one or more TPR bead populations in amounts sufficient to provide a pharmacokinetic profile suitable for a once-daily dosing regimen in patients in need of such a medication,
optionally wherein the pharmaceutical multiparticulate dosage form comprises therapeutically effective amounts of two or more TPR bead populations, wherein each of the two or more TPR bead populations exhibit differing release characteristics following a pre-determined lag-time.

13. The method of claim 12, wherein each of said coating or applying steps comprises coating or applying from a solution in a pharmaceutically acceptable solvent system or from an aqueous dispersion.

14. The method of claim 12, wherein step (f) is compressing the beads into an orally disintegrating tablet, optionally compressing on a rotary tablet press equipped with an external lubrication system to lubricate the dies and punches prior to compression
and wherein said method further comprises:
g) optionally taste-masking the one or more bead populations by solvent coacervation or by fluid-bed coating
h) granulating a sugar alcohol or a saccharide, or a combination thereof, and a disintegrant, each having an average particle size of not more than about 30 µm to produce rapidly dispersing microgranules with an average particle size of not more than about 400 µm;
i) blending the one or more bead populations with the rapidly dispersing microgranules, wherein the ratio of one or more bead populations to rapidly dispersing microgranules optionally ranges from about 1:6 to about 1:2; and
j) compressing the blend of step (i) into orally disintegrating tablets using a rotary tablet press,

## Patentansprüche

1. Pharmazeutische multipartikuläre Dosierform, die eine oder mehrere Populationen aus zeitlich abgestimmten, impulsartigen Freisetzungs- (TPR-) Kügelchen eines schwach basischen Arzneimittels umfasst,
worin das schwach basische Arzneimittel ein selektives, Stickstoff- (N) haltiges Serotonin-5-HT₃-Blockiermittel oder ein pharmazeutisch annehmbares Salz davon mit einem pKa im Bereich von etwa 5 bis 14 und einer Löslichkeit von nicht mehr als etwa 200 µg/ml bei pH 6,8 umfasst;
worin die TPR-Kügelchen Kernpartikel aus organischer Säure umfassen, die zumindest eine pharmazeutisch annehmbare organische Säure umfassen, die mit einer SR- (Retard-) Polymermembran umfassend ein wasserunlösliches Polymer allein oder in Kombination mit einem wasserlöslichen Polymer oder einem magensaftresistenten Polymer beschichtet sind, auf der das schwach basische Arzneimittel geschichtet ist und das ferner mit einer Latenzzeitmembran beschichtet ist, sodass die organische Säure sowie das schwach basische Arzneimittel vergleichbare Arzneimittel-Freisetzungsprofile aufweisen; und
worin das schwach basische Arzneimittel und die organische Säure in der Do-sierform nicht miteinander in Kontakt kommen.

2. Pharmazeutische multipartikuläre Dosierform nach Anspruch 1, worin das schwach basische Arzneimittel bei pH 6,8 ein Verhältnis einer optimalen Höchstdosis zur Löslichkeit von nicht weniger als etwa 100 aufweist und worin zumindest eine pharmazeutisch annehmbare organische Säure das schwach basische Arzneimittel vor dessen Freisetzung in eine feindliche Umgebung im Verdauungstrakt solubilisiert, worin das schwach basische Arzneimittel praktisch unlöslich ist, und die Dosierform 24 h nach der für ein einmal täglich verabreichtes Dosierregime geeigneten Dosierung für Patienten mit Bedarf daran ein Pharmakokinetik-Profil aufweist.

3. Pharmazeutische multipartikuläre Dosierform nach Anspruch 1, worin die TPR-Kügelchen eine äußere Latenzzeitbeschichtung mit einem wasserunlöslichen Polymer in Kombination mit einem magensaftresistenten Polymer umfassen, wobei die äußere Latenzzeitbeschichtung eine Verzögerungszeit von etwa 2 bis etwa 7 h bereitstellt, bevor das schwach basische Arzneimittel freigesetzt wird;
worin die äußere Latenzzeitbeschichtung über einer Retard- (SR-) Beschichtung umfassend ein wasserunlösliches Polymer allein oder in Kombination mit einem wasserlöslichen Polymer angeordnet ist, wobei die SR-Beschichtung ein Profil mit verzögerter Freisetzung bereitstellt,
worin die SR-Beschichtung über der Schicht aus schwach basischem Arzneimittel angeordnet ist; und
worin die Schicht des schwach basischen Arzneimittels über der inneren Barrierebeschichtung angeordnet ist, die über den organische Säure enthaltenden Kernen angeordnet ist, wobei die innere Barrierebeschichtung ein Retard-Profil bereitstellt.

4. Pharmazeutische multipartikuläre Dosierform nach Anspruch 1 in der Form einer Schmelztablette (ODT), worin gegebenenfalls
(a) die ODT ferner schnell zerfallendes Mikrogranulat umfasst, das ein Sprengmittel und einen Zuckeralkohol oder ein Saccharid oder eine Kombination daraus umfasst und worin jedes des Sprengmittels und des Zuckeralkohols oder des Saccharids eine mittlere Partikelgröße von nicht mehr als etwa 30 µm aufweist,
(b) das Mikrogranulat eine mittlere Partikelgröße von nicht mehr als 400 µm aufweist und
(c) die ODT gegebenenfalls eine Zerfallszeit von etwa 60 Sekunden oder weniger bei Kontakt mit Speichel in der Mundhöhle aufweist.

5. Pharmazeutisch multipartikuläre Dosierform nach einem der Ansprüche 1 bis 4, worin das schwach basische Arzneimittel Ondansetronhydrochlorid ist und die pharmazeutisch annehmbare organische Säure Fumarsäure ist.

6. Pharmazeutisch multipartikuläre Dosierform nach einem der vorangegangenen Ansprüche, worin das schwach basische Arzneimittel Ondansetron oder ein pharmazeutisch annehmbares Salz davon mit einem pKa von 7,4 und einer Löslichkeit von weniger als 100 µg/ml bei einem pH von 6,8 ist.

7. Pharmazeutische multipartikuläre Dosierform nach Anspruch 1, worin
(a) die organische Säure aus der Gruppe bestehend aus Citronensäure, Fumarsäure, Apfelsäure, Maleinsäure, Weinsäure, Bernsteinsäure, Oxalsäure, Asparaginsäure, Glutaminsäure und Gemischen daraus ausgewählt ist;
(b) das Gewichtsverhältnis des schwach basischen Arzneimittels zu der organischen Säure von etwa 5:1 bis 1:10 variiert, um Pharmakokinetik-Zielprofile, die für ein einmal täglich zu verabreichendes Dosierregime geeignet sind, bereitzustellen; oder
(c) die organische Säure nicht bis zum Abschluss der Freisetzung des schwach basischen Arzneimittels aus der Dosierform aufgebraucht ist, wenn die Auflösung mittels der US-Pharmakopöe- (USP-) Auflösungsmethodologie unter Verwendung eines Zweistufen-Auflösungsmediums (zuerst 2 h lang in 0,1 N HCl, gefolgt von Testen in einem Puffer bei pH 6,8) getestet wird.

8. Pharmazeutische multipartikuläre Dosierform nach Anspruch 1 oder Anspruch 3, worin die Kernpartikel aus organischer Säure Folgendes umfassen:
i) einen Kristall aus organischer Säure;
ii) einen inerten Partikel, der mit einer organischen Säure und einem Polymerbinder beschichtet ist; oder
iii) ein Pellet oder eine Mikrotablette, die die organische Säure, einen Polymerbinder und ein Verdünnungs-/Auffüllmittel umfassen, das/die durch Rotogranulation, Granulation-Extrusion-Sphäronisation oder Granulations-Kompression hergestellt wurde.

9. Pharmazeutische multipartikuläre Dosierform nach Anspruch 3, worin
(a) die SR-Beschichtung ein wasserunlösliches Polymer allein oder ein wasserunlösliches Polymer in Kombination mit einem wasserlöslichen Polymer in einem Verhältnis von etwa 9:1 bis 5:5 umfasst und worin die SR-Beschichtung für eine Gewichtserhöhung von etwa 1,5 Gew.-% bis 20 Gew.-%, bezogen auf das Gesamtgewicht der SR-beschichteten Kernpartikel aus organischer Säure, aufgebracht wird,
(b) die Latenzzeitbeschichtung ein wasserunlösliches Polymer in Kombination mit einem magensaftresistenten Polymer in einem Verhältnis von jeweils etwa 9:1 bis 1:3 für eine Gewichtserhöhung von etwa 10 Gew.-% bis 60 Gew.-%, bezogen auf das Trockengewicht der TPR-Kügelchen, umfasst,
(c) die Latenzzeitbeschichtung Ethylcellulose mit einer mittleren Viskosität von 10 cps und Hydroxypropylmethylcellulosephthalat umfasst und gegebenenfalls ferner einen Weichmacher umfasst oder
(d) die Latenzzeitbeschichtung Ethylcellulose mit einer mittleren Viskosität von 10 cps und Hydroxypropylmethylcellulosephtahalat umfasst und das schwach basische Arzneimittel Ondansetron oder ein pharmazeutisch annehmbares Salz davon mit einem pKa von 7,4 und einer Löslichkeit von weniger als 100 µg/ml bei einem pH von 6,8 ist.

10. Pharmazeutische multipartikuläre Dosierform nach Anspruch 1, worin
(a) das wasserunlösliche Polymer aus der Gruppe bestehend aus Ethylcellulose, Celluloseacetat, Celluloseacetatbutyrat, Polyvinylacetat, neutralen Methacrylsäure-Methylmethacrylat-Copolymeren und Gemischen daraus ausgewählt ist,
(b) das wasserlösliche Polymer aus der Gruppe bestehend aus Methylcellulose, Hydroxypropylmethylcellulose, Hydroxypropylcellulose, Polyvinylpyrrolidon und Polyethylenglykol und Gemischen daraus ausgewählt ist,
(c) das magensaftresistente Polymer aus der Gruppe bestehend aus Celluloseacetatphthalat, Hydroxypropylmethylcellulosephthalat, Hydroxypropylmethylcellulosesuccinat, Polyvinylacetatphthalat, pH-empfindlichen Methacrylsäure-Methylmethacrylat-Copolymeren, Schellack, Derivaten davon und Gemischen daraus ausgewählt ist und/oder
(d) zumindest eine der SR-Beschichtung, der inneren Barrierebeschichtung und der äußeren Latenzzeitbeschichtung ferner einen Weichmacher umfasst, der aus der Gruppe bestehend aus Triacetin, Tributylcitrat, Triethylcitrat, Acetyltri-n-butylcitrat, Diethylphthalat, Dibutylsebacat, Polyethylenglykol, Polypropylenglykol, Ricinusöl, acetylierten Mono- und Diglyzeriden und Gemischen daraus ausgewählt ist.

11. Pharmazeutische multipartikuläre Dosierform nach Anspruch 1 oder Anspruch 3, worin das schwach basische Arzneimittel Ondansetron oder ein pharmazeutisch annehmbares Salz davon umfasst; wobei jede TPR-Kügelchenpopulation SR-beschichtete Kernpartikel aus organischer Säure umfassend Fumarsäure umfasst; und die Latenzzeitbeschichtung Ethylcellulose und Hydroxypropylmethylcellulosephthalat in einem Verhältnis von etwa 9:1 bis etwa 1:3 für eine Gewichtserhöhung von 5 % bis 60 % umfasst, wobei jede Population von TPR-Kügelchen bei oraler Verabreichung der Dosierform eine vorbestimmte Latenzzeit und verschiedene Freisetzungscharakteristika aufweist.

12. Verfahren zur Herstellung der pharmazeutischen multipartikulären Dosierform, die eine oder mehrere Populationen aus zeitlich abgestimmten, impulsartigen Freisetzungs- (TPR-) Kügelchen eines selektiven, schwach basischen Stickstoff- (N) haltigen Serotonin-5-HT₃-Blockiermittels oder eines pharmazeutisch annehmbaren Salzes davon mit einem pKa im Bereich von etwa 5 bis 14 und einer Löslichkeit bei pH 6,8 von nicht mehr als etwa 200 µg/ml und einem Verhältnis der optimalen Höchstdosis zur Löslichkeit bei pH 6,8 von nicht weniger als etwa 100 und zumindest eine pharmazeutisch annehmbare organische Säure als Auflösungsmittel umfasst, welches Folgendes umfasst:
a) Herstellen von Kernpartikeln aus organischer Säure umfassend zumindest eine pharmazeutisch annehmbare organische Säure;
b) Herstellen von SR-beschichteten Kernpartikeln aus organischer Säure durch Beschichten der Kernpartikel aus organischer Säure mit einer SR-Beschichtung, die ein wasserunlösliches Polymer allein oder ein wasserunlösliches Polymer in Kombination mit einem wasserlöslichen Polymer oder einem magensaftresistenten Polymer in einem Verhältnis von etwa 95:5 bis etwa 50:50 für eine Gewichtserhöhung von bis zu etwa 20 % umfasst, um ein Retard-Freisetzungsprofil bereitzustellen;
c) Herstellen von IR- (Sofortfreisetzungs-) Kügelchen durch Beschichten einer Lösung, die das schwach basische Arzneimittel oder ein pharmazeutisch annehmbares Salz davon und einen Polymerbinder umfasst, und gegebenenfalls Aufbringen einer Schutzbeschichtung umfassend ein wasserlösliches Polymer auf die mit der inneren Barriere beschichteten Kernpartikel aus organischer Säure;
d) gegebenenfalls Herstellen von SR-Kügelchen durch Aufbringen einer SR-(Barriere-) Beschichtung umfassend ein wasserunlösliches Polymer allein oder ein wasserunlösliches Polymer in Kombination mit einem wasserlöslichen Polymer in einem Verhältnis von etwa 95:5 bis etwa 50:50 auf die IR-Kügelchen für eine Gewichtszunahme von etwa 1,5 % bis 20 % des gesamten Trockengewichts der beschichteten SR-Kügelchen;
e) Herstellen von TPR-Kügelchen durch Aufbringen einer äußeren Latenzzeitbeschichtung umfassend ein wasserunlösliches Polymer in Kombination mit einem magensaftresistenten Polymer auf die Kügelchen aus Schritt (c) oder (d) in einem Verhältnis von etwa 9:1 bis 1:3 für eine Gewichtserhöhung von etwa 10 % bis 60 % des gesamten Trockengewichts der TPR-Kügelchen; und
f) Einfüllen in eine Kapsel oder Verpressen zu einer Schmelztablette von einer oder mehreren TPR-Kügelchenpopulationen in ausreichenden Mengen, um ein Pharmakokinetik-Profil bereitzustellen, das für ein einmal täglich zu verabreichendes Do-sierregime in Patienten mit Bedarf an einer solchen Medikation geeignet ist, gegebenenfalls worin die pharmazeutische multipartikuläre Dosierform therapeutisch wirksame Mengen zweier oder mehrerer TPR-Kügelchenpopulationen umfasst, worin jede der zwei oder mehreren TPR-Kügelchenpopulationen nach einer vorbestimmten Latenzzeit unterschiedliche Freisetzungscharakteristika aufweist.

13. Verfahren nach Anspruch 12, worin jeder der Beschichtungs- und Aufbringungsschritte das Beschichten oder Aufbringen aus einer Lösung in einem pharmazeutisch annehmbaren Lösungsmittelsystem oder aus einer wässrigen Dispersion umfasst.

14. Verfahren nach Anspruch 12, worin Schritt (f) das Pressen der Kügelchen in eine Schmelztablette ist, gegebenenfalls Pressen auf einer Rotationstablettenpresse, die mit einem externen Schmiersystem ausgestattet ist, um die Pressstempel und Patrizen vor dem Pressen zu schmieren,
und worin das Verfahren ferner Folgendes umfasst:
g) gegebenenfalls Geschmacksmaskieren der einen oder mehrerer der Kügelchenpopulationen durch Lösungsmittel-Koazervierung oder durch Fließbettbeschichten;
h) Granulieren eines Zuckeralkohols oder eines Saccharids oder einer Kombination daraus und eines Sprengmittels, wobei jedes davon eine mittlere Partikelgröße von nicht mehr als etwa 30 µm aufweist, um ein schnell zerfallendes Mikrogranulat mit einer mittleren Partikelgröße von nicht mehr als etwa 400 µm herzustellen;
i) Vermischen der einen oder mehrerer der Kügelchenpopulationen mit dem schnell zerfallenden Mikrogranulat, worin das Verhältnis von der einen oder mehrerer der Kügelchenpopulationen zu schnell zerfallendem Mikrogranulat gegebenenfalls im Bereich von etwa 1:6 bis etwa 1:2 liegt; und
j) Pressen der Mischung aus Schritt (i) in sich im Mund zersetzende Tabletten unter Verwendung einer Rotationstablettenpresse.

## Revendications

1. Forme pharmaceutique multiparticulaire comprenant une ou plusieurs populations de billes à libération temporisée, pulsée (TPR) d'un médicament faiblement basique ; **caractérisée en ce que** le médicament faiblement basique comprend un agent bloquant la sérotonine 5-HT₃ sélectif contenant de l'azote (N), ou un sel pharmaceutiquement acceptable de celui-ci, ayant un pKa dans la plage d'environ 5 à 14 et une solubilité de pas plus d'environ 200 µg/ml à pH 6,8 ;
où les billes TPR comprennent des particules de noyau d'acide organique comprenant au moins un acide organique pharmaceutiquement acceptable, qui sont pourvues d'un enrobage de barrière avec une membrane de polymère SR (libération prolongée) comprenant un polymère insoluble dans l'eau seul ou en combinaison avec un polymère hydrosoluble ou un polymère entérique, sur lesquelles le médicament faiblement basique est couché et en outre enrobé avec une membrane à temps de latence de sorte que l'acide organique et le médicament faiblement basique présentent des profils de libération de médicament comparables ; et
dans laquelle le médicament faiblement basique et l'acide organique n'entrent pas en contact l'un avec l'autre dans la forme pharmaceutique.

2. Forme pharmaceutique multiparticulaire de la revendication 1, dans laquelle le médicament faiblement basique a un rapport de la dose maximale optimale à la solubilité à pH 6,8 d'au moins environ 100, et au moins un acide organique pharmaceutiquement acceptable solubilise ledit médicament faiblement basique avant de le libérer dans un environnement intestinal hostile dans lequel ledit médicament faiblement basique est pratiquement insoluble, et ladite forme pharmaceutique présente un profil pharmacocinétique à 24 heures post-administration adapté pour un régime posologique une fois par jour pour des patients nécessitant cela.

3. Forme pharmaceutique multiparticulaire de la revendication 1, dans laquelle lesdites billes TPR comprennent un enrobage externe à libération retardée comprenant un polymère insoluble dans l'eau en combinaison avec un polymère entérique, ledit enrobage externe à libération retardée produisant un temps de latence d'environ 2 à environ 7 heures avant le début de la libération du médicament faiblement basique ;
dans laquelle ledit enrobage à libération retardée est disposé sur un enrobage à libération prolongée (SR) comprenant un polymère insoluble dans l'eau seul ou en combinaison avec un hydrosoluble, ledit enrobage SR présentant un profil de libération prolongée,
dans laquelle ledit enrobage SR est disposé sur la couche de médicament faiblement basique ; et dans laquelle la couche de médicament faiblement basique est disposée sur l'enrobage de barrière interne, qui est disposé sur les noyaux contenant un acide organique, ledit enrobage de barrière interne produisant un profil de libération prolongée.

4. Forme pharmaceutique multiparticulaire de la revendication 1, sous la forme d'un comprimé à désintégration par voie buccale (ODT), dans lequel, facultativement,
(a) ledit ODT comprend en outre des microgranules se dispersant rapidement, qui comprennent un délitant et un polyol ou un saccharide ou une combinaison de ceux-ci, et chacun du délitant et du polyol ou saccharide ont une taille de particule moyenne de pas plus d'environ 30 µm,
(b) lesdits microgranules ont une taille de particule moyenne de pas plus de 400 µm, et
(c) ledit ODT a facultativement un temps de désintégration d'environ 60 secondes ou moins lors du contact avec de la salive dans la cavité buccale.

5. Forme pharmaceutique multiparticulaire de l'une quelconque des revendications 1 à 4, dans laquelle le médicament faiblement basique est le chlorhydrate d'ondansétron et l'acide organique pharmaceutiquement acceptable est l'acide fumarique.

6. Forme pharmaceutique multiparticulaire de l'une quelconque des revendications précédentes, dans laquelle ledit médicament faiblement basique est l'ondansétron, ou un sel pharmaceutiquement acceptable de celui-ci ayant un pKa de 7,4 et une solubilité inférieure à 100 µg/ml à un pH de 6,8.

7. Forme pharmaceutique multiparticulaire de la revendication 1, dans laquelle
(a) l'acide organique est choisi dans le groupe constitué de l'acide citrique, l'acide fumarique, l'acide malique, l'acide maléique, l'acide tartrique, l'acide succinique, l'acide oxalique, l'acide aspartique, l'acide glutamique, et des mélanges de ceux-ci ;
(b) le rapport du médicament faiblement basique à l'acide organique varie d'environ 5:1 à 1:10 en poids, pour produire des profils pharmacocinétiques cibles adaptés pour un régime posologique une fois par jour ; ou
(c) l'acide organique n'est pas déplété depuis ladite forme pharmaceutique jusqu'à la libération totale du médicament faiblement basique depuis la forme pharmaceutique lorsque la dissolution est testée par la méthodologie de dissolution de la United States Pharmacopoeia (USP) utilisant un milieu de dissolution à deux étapes (2 premières heures dans HCl 0,1 N suivies par essai dans un tampon à pH 6,8).

8. Forme pharmaceutique multiparticulaire de la revendication 1 ou la revendication 3, dans laquelle lesdites particules de noyau d'acide organique comprennent :
i) un cristal d'acide organique ;
ii) une particule inerte revêtue avec un acide organique et un liant polymère ; ou
iii) une pilule ou un micro-comprimé comprenant l'acide organique, un liant polymère et un diluant/charge, préparé par rotogranulation, granulation-extrusion-sphéronisation ou granulation-compression.

9. Forme pharmaceutique multiparticulaire de la revendication 3, dans laquelle
(a) l'enrobage SR comprend un polymère insoluble dans l'eau seul, ou un polymère insoluble dans l'eau en combinaison avec un polymère hydrosoluble à un rapport d'environ 9:1 à 5:5, et où ledit enrobage SR est appliqué pour un gain de poids d'environ 1,5 % à 20 % en poids sur la base du poids total des particules de noyau d'acide organique avec enrobage SR
(b) ledit enrobage à libération retardée comprend un polymère insoluble dans l'eau en combinaison avec un polymère entérique à un rapport d'environ 9:1 à 1:3, respectivement, pour un gain de poids d'environ 10 % à 60 % en poids sur la base du poids sec des billes TPR,
(c) l'enrobage à libération retardée comprend de l'éthylcellulose ayant une viscosité moyenne de 10 cps et du phtalate d'hydroxypropylméthylcellulose et comprend facultativement en outre un plastifiant, ou
(d) l'enrobage à libération retardée comprend de l'éthylcellulose ayant une viscosité moyenne de 10 cps et du phtalate d'hydroxypropylméthylcellulose et le médicament faiblement basique est l'ondansétron ou un sel pharmaceutiquement acceptable de celui-ci ayant un pKa de 7,4 et une solubilité inférieure à 100 µg/ml à un pH de 6,8.

10. Forme pharmaceutique multiparticulaire de la revendication 1, dans laquelle
(a) le polymère insoluble dans l'eau est choisi dans le groupe constitué de l'éthylcellulose, l'acétate de cellulose, l'acétate-butyrate de cellulose, le polyacétate de vinyle, des copolymères d'acide méthacrylique-méthacrylate de méthyle neutre, et des mélanges de ceux-ci
(b) le polymère hydrosoluble est choisi dans le groupe constitué de la méthylcellulose, l'hydroxypropylméthylcellulose, l'hydroxypropylcellulose, la polyvinylpyrrolidone et le polyéthylèneglycol, et des mélanges de ceux-ci
(c) le polymère entérique est choisi dans le groupe constitué de l'acétate-phtalate de cellulose, le phtalate d'hydroxypropylméthylcellulose, le succinate d'hydroxypropylméthylcellulose, l'acétate-phtalate de polyvinyle, des copolymères d'acide méthacrylique-méthacrylate de méthyle sensible au pH, la gomme laque, des dérivés de ceux-ci, et des mélanges de ceux-ci, et/ou
(d) au moins l'un du revêtement SR, l'enrobage de barrière interne et l'enrobage à libération retardée externe comprend en outre un plastifiant choisi dans le groupe constitué de la triacétine, le citrate de tributyle, le citrate de triéthyle, le citrate de tri-n-butyl-acétyle, le phtalate de diéthyle, le sébacate de dibutyle, le polyéthylèneglycol, le polypropylèneglycol, l'huile de ricin, des mono- et di-glycérides acétylés, et des mélanges de ceux-ci.

11. Forme pharmaceutique multiparticulaire de la revendication 1 ou la revendication 3, dans laquelle ledit médicament faiblement basique comprend l'ondansétron ou un sel pharmaceutiquement acceptable de celui-ci ; chaque populations de billes TPR comprend des particules de noyau d'acide organique avec enrobage SR comprenant de l'acide fumarique ; et l'enrobage à libération retardée comprend l'éthylcellulose et le phtalate d'hydroxypropylméthyl-cellulose à un rapport d'environ 9:1 à environ 1:3, pour un gain de poids de 5 % à 60 %, chaque population de billes TPR présentant, après administration orale de la forme pharmaceutique, un temps de latence prédéterminé et des caractéristiques de libération différentes.

12. Procédé de préparation de la forme pharmaceutique multiparticulaire comprenant une ou plusieurs populations de billes à libération temporisée, pulsée (TPR) d'un agent bloquant la sérotonine 5-HT3 sélectif contenant de l'azote (N), ou un sel pharmaceutiquement acceptable de celui-ci, ayant un pKa dans la plage d'environ 5 à 14 et une solubilité de pas plus d'environ 200 µg/ml à pH 6,8 et un rapport de la dose maximale optimale à la solubilité à pH 6,8 d'au moins environ 100, et au moins un acide organique pharmaceutiquement acceptable en tant que solubilisant comprenant :
a) la préparation de particules de noyau d'acide organique comprenant au moins un acide organique pharmaceutiquement acceptable ;
b) la préparation de particules de noyau d'acide organique avec enrobage SR par enrobage des particules de noyau d'acide organique avec un enrobage SR comprenant un polymère insoluble dans l'eau seul, ou un polymère insoluble dans l'eau en combinaison avec un polymère hydrosoluble ou un polymère entérique à un rapport d'environ 95:5 à environ 50:50 pour un gain de poids de jusqu'à environ 20 %, pour produire un profil de libération prolongée ;
c) la préparation de billes IR (libération immédiate) par enrobage d'une solution comprenant le médicament faiblement basique, ou un sel pharmaceutiquement acceptable de celui-ci, et un liant de polymère, et facultativement l'application d'une couche de scellage protectrice comprenant un polymère hydrosoluble, sur les particules de noyau d'acide organique avec enrobage de barrière interne ;
d) facultativement la préparation de billes SR par application d'un enrobage SR (barrière) comprenant un polymère insoluble dans l'eau seul, ou un polymère insoluble dans l'eau en combinaison avec un polymère hydrosoluble à un rapport d'environ 95:5 à environ 50:50 sur les billes IR, pour un gain de poids d'environ 1,5 % à 20 % du poids sec total des billes SR enrobées ;
e) la préparation de billes TPR par application d'un enrobage à libération retardée externe comprenant un polymère insoluble dans l'eau en combinaison avec un polymère entérique sur les billes de l'étape (c) ou (d), à un rapport d'environ 9:1 à 1:3 pour un gain de poids d'environ 10 % à 60 % du poids sec total des billes TPR ; et
f) le remplissage dans une capsule ou la compression en comprimé ou comprimé à désintégration par voie buccale, une ou plusieurs populations de billes TPR dans des quantités suffisantes pour produire un profil pharmacocinétique adapté pour un régime posologique une fois par jour chez des patients nécessitant une telle médication,
facultativement dans lequel la forme pharmaceutique multiparticulaire comprend des quantités thérapeutiquement efficaces de deux populations de billes TPR ou plus, où chacune des deux populations de billes TPR ou plus présente des caractéristiques de libération différentes après un temps de latence prédéterminé.

13. Procédé de la revendication 12, dans lequel chacune desdites étapes d'enrobage ou d'application comprend l'enrobage ou l'application d'une solution dans un système de solvant pharmaceutiquement acceptable ou d'une dispersion aqueuse.

14. Procédé de la revendication 12, dans lequel l'étape (f) est la compression des billes en comprimé à désintégration par voie buccale, facultativement la compression sur une presse de fabrication de comprimés rotative équipée d'un système de lubrification externe pour lubrifier les matrices et les poinçons avant compression et **caractérisé en ce que** ledit procédé comprend en outre :
g) facultativement le masquage de goût des une ou plusieurs populations de billes par coacervation de solvant ou par enrobage sur lit fluidisé
h) granulation d'un polyol ou d'un saccharide, ou une combinaison de ceux-ci, et un délitant, ayant chacun une taille de particule moyenne de pas plus d'environ 30 µm pour produire des microgranules se dispersant rapidement avec une taille de particule moyenne de pas plus d'environ 400 µm ;
i) mélange des une ou plusieurs populations de billes avec les microgranules se dispersant rapidement, où le rapport d'une ou plusieurs populations de billes aux microgranules se dispersant rapidement est facultativement dans la plage d'environ 1:6 à environ 1:2 ; et
j) la compression du mélange de l'étape (i) en comprimés à désintégration par voie buccale en utilisant une presse de fabrication de comprimés rotative.
